# EUROPÄISCHE PATENTSCHRIFT

(11) **EP 2 792 345 B1**
(45) Veröffentlichungstag und Bekanntmachung des Hinweises auf die Patenterteilung: **09.10.2019**
(21) Anmeldenummer: 13163830.6
(22) Anmeldetag: 15.04.2013
(51) Int. Cl.: A61K 6/027, C03C 3/097, C03C 3/118, C03C 4/00, C03C 10/00

(54) **Lithiumsilikat-Glaskeramik und -Glas mit Gehalt an Cäsiumoxid**
Lithium silicate glass ceramic and glass with caesium oxide content
Vitrocéramique et verre au lithium-silice ayant une teneur en oxyde de césium

(43) Veröffentlichungstag der Anmeldung: 22.10.2014
(73) Patentinhaber: Ivoclar Vivadent AG, 9494 Schaan (LI)
(72) Erfinder: Rampf, Markus, 8853 Lachen (CH); Dittmer, Marc, 6800 Feldkirch (AT); Ritzberger, Christian, 9472 Grabs (CH); Höland, Wolfram, 9494 Schaan (LI); Bolle, Urs, 6842 Koblach (AT); Schweiger, Marcel, 7000 Chur (CH); Rheinberger, Volker, 9490 Vaduz (LI)
(74) Vertreter: Uexküll & Stolberg

(56) Entgegenhaltungen:
- EP-A1- 2 377 831
- WO-A1-2013/086187
- WO-A2-2013/053863

## Beschreibung

Die Erfindung betrifft Lithiumsilikat-Glaskeramik und Lithiumsilikatglas, die Cäsiumoxid enthalten und sich durch einen in einem breiten Bereich von insbesondere 8,0 bis 17,0·10⁻⁶ K⁻¹ einstellbaren linearen thermischen Ausdehnungskoeffizienten auszeichnen und deshalb vor allem im Dentalbereich zur Verblendung von Oxidkeramikrestaurationen und von Metallrestaurationen eignen.

In der Zahnheilkunde werden Dentalrestaurationen üblicherweise mit keramischen Schichten verblendet, um das Aussehen der Restauration an das der natürlichen Zähne anzugleichen. Solche verblendeten Restaurationen werden auch als Verblendkeramiken bezeichnet. Um Spannungen zwischen dem zu verblendenden Restaurationsmaterial und der Keramikschicht zu vermeiden, ist es erforderlich, dass die Wärmeausdehnungskoeffizienten der keramischen Werkstoffe an die des Restaurationsmaterials angepasst sind.

Zur Beschichtung oder Verblendung von Oxidkeramiken, wie etwa Zirkonoxidkeramiken, sind in der Vergangenheit bereits Glaskeramiken eingesetzt worden. Dazu zählen auf Feldspat basierende Keramiken oder Fluoroapatit-Glaskeramiken.

Weiter sind Lithiumdisilikat-Glaskeramiken bekannt, die aufgrund ihrer hohen Transluzenz und sehr guten mechanischen Eigenschaften besonders im Dentalbereich und dabei vornehmlich zur Herstellung von Dentalkronen und kleinen Brücken Anwendung finden. Die bekannten Lithiumsilikat-Glaskeramiken enthalten üblicherweise als Hauptkomponenten SiO₂, Li₂O, Al₂O₃, Na₂O oder K₂O und Keimbildner wie P₂O₅.

EP 0 885 855 A2 und EP 0 885 856 A2 beschreiben apatithaltige Glaskeramiken mit ausgezeichneten optischen und chemischen Eigenschaften und einer Festigkeit im Bereich von 110 MPa, die sich zur Verblendung von ZrO₂-Gerüsten eignen.

WO 2004/021921 A1 beschreibt ein Glas zur Verblendung von ZrO₂, welches jedoch nur geringe Festigkeit aufweist.

EP 1 253 116 A1 beschreibt eine Mischung aus einem Lithiumsilikatglas mit Leucitkristallen zur Verblendung von Metallgerüsten. Auch dieses Glas weist nur eine ungenügende Festigkeit auf.

WO 2012/082156 A1 beschreibt ein Lithiumsilikat-Produkt zur Verblendung von Metallgerüsten mit einem Ausdehnungskoeffizienten WAK_{100-400°C} von 12 bis 13,5·10⁻⁶ K⁻¹ und Festigkeiten von bis zu 300 MPa.

EP 2 377 831 A1 beschreibt eine Lithiumsilikat-Glaskeramik mit Gehalten an ZrO₂. Der Ausdehnungskoeffizient der Glaskeramik ist nicht für die Verblendung von Metallgerüsten geeignet.

Damit eine Dentalglaskeramik zum Verblenden der gesamten Bandbreite der üblicherweise verwendeten Restaurationsmaterialien, wie Dentalmetallen und -legierungen bis hin zu Oxidkeramiken eingesetzt werden kann, ist es erforderlich, dass ihr Ausdehnungskoeffizient in einem breiten Bereich einstellbar ist. Zudem müssen die Glaskeramiken hohen Anforderungen in Bezug auf ihre optischen und mechanischen Eigenschaften genügen und insbesondere eine sehr hohe Festigkeit aufweisen.

Bekannte Glaskeramiken und Gläser erfüllen die Forderung nach in einem weiten Bereich einstellbaren thermischen Ausdehnungskoeffizienten und ausreichender Festigkeit häufig nicht. Weiter sind bei den bekannten Glaskeramiken regelmäßig das Erdalkalimetalloxid BaO sowie die Alkalimetalloxide K₂O und/oder Na₂O als essentielle Komponenten vorhanden, die dort zur Erzeugung der Glaskeramiken und insbesondere der Bildung der meist angestrebten Lithiumdisilikat-Hauptkristallphase offenbar erforderlich sind.

Es besteht daher ein Bedarf an Lithiumsilikat-Glaskeramiken, bei denen der lineare thermische Ausdehnungskoeffizient WAK_{100-400°C} über einen breiten Bereich und insbesondere im Bereich von 8,0 bis 17,0·10⁻⁶ K⁻¹ und vorzugsweise im Bereich von 8,8 bis 16,7·10⁻⁶ K⁻¹ einstellbar ist. Weiter sollen sie auch ohne die bisher als erforderlich angesehenen Alkalimetalloxide K₂O oder Na₂O sowie insbesondere ohne das Erdalkalimetalloxid BaO herstellbar sein und sich aufgrund vor allem ihrer optischen und mechanischen Eigenschaften insbesondere zur Verblendung von dentalen Restaurationen einschließlich Oxidkeramikrestaurationen und Metallrestaurationen eignen.

Diese Aufgabe wird durch die Verwendung einer Lithiumsilikat-Glaskeramik oder eines Lithiumsilikatglases nach einem der Ansprüche 1 bis 16 gelöst. Gegenstand der Erfindung sind ebenfalls das Verfahren nach den Ansprüchen 17 bis 23, der Verbundwerkstoff nach Anspruch 24, die Lithiumsilikat-Glaskeramik nach den Ansprüchen 25 und 26 und das Lithiumsilikatglas nach Anspruch 27.

Die erfindungsgemäße Verwendung zeichnet sich dadurch aus, dass eine Lithiumsilikat-Glaskeramik oder ein Lithiumsilikatglas, die die folgenden Komponenten enthalten

| Komponente | Gew.-% | | |
|---|---|---|---|
| SiO₂ | 54,0 | bis | 78,0 |
| Li₂O | 11,8 | bis | 19,0 |
| Cs₂O | 4,4 | bis | 13,0 |
| Al₂O₃ | 1,7 | bis | 9,0 |
| P₂O₅ | 0,5 | bis | 9,0, |

zur Beschichtung eines Substrats ausgewählt aus Metallen und Legierungen verwendet werden.

Es hat sich überraschend gezeigt, dass die erfindungsgemäße Lithiumsilikat-Glaskeramik einen linearen thermischen Ausdehnungskoeffizienten WAK_{100-400°C} aufweist, der leicht in einem breiten Bereich von insbesondere 8,0 bis 17,0·10⁻⁶ K⁻¹ und vorzugsweise 8,8 bis 16,7·10⁻⁶ K⁻¹ einstellbar ist, und zudem hervorragende optische und mechanische Eigenschaften wie hohe Festigkeit und Bruchzähigkeit aufweist. Diese Glaskeramik ist daher sowohl zur Beschichtung von Oxidkeramiken als auch von Metallen und Legierungen geeignet. Besonders überraschend ist, dass dabei die Bildung einer Glaskeramik mit Lithiummeta- und/oder -disilikat als Hauptkristallphase auch bei Abwesenheit verschiedener bei konventionellen Glaskeramiken als erforderlich angesehener Komponenten wie insbesondere K₂O, Na₂O und BaO gelingt. Die Bildung der erfindungsgemäßen Glaskeramik kann auch durch die Verwendung des erfindungsgemäßen Lithiumsilikatglases erzielt werden, welches einen Vorläufer für die Lithiumsilikat-Glaskeramik darstellt und vor, während oder nach dem Aufbringen auf das Substrat in diese umgewandelt werden kann.

Es ist bevorzugt, dass die erfindungsgemäß verwendete Lithiumsilikat-Glaskeramik und das erfindungsgemäß verwendete Lithiumsilikatglas mindestens eine und bevorzugt alle der folgenden Komponenten in den angegebenen Mengen enthalten

| Komponente | Gew.-% | | | |
|---|---|---|---|---|
| SiO₂ | 55,1 | bis | 77,1 | |
| Li₂O | 11,8 | bis | 17,8 | |
| Cs₂O | 4,4 | bis | 11,7 | |
| Al₂O₃ | 1,7 | bis | 8,0 | |
| P₂O₅ | 1,3 | bis | 7,5 | |
| ZrO₂ | 0 | bis | 4,5, | insbesondere 0 bis 4,0 |
| Übergangsmetalloxid | 0 | bis | 7,5, | insbesondere 0 bis 7,0, |

wobei das Übergangsmetalloxid ausgewählt ist aus der Gruppe bestehend aus Oxiden von Yttrium, Oxiden von Übergangsmetallen mit Ordnungszahl 41 bis 79 und Mischungen dieser Oxide.

Vorzugsweise enthalten die Lithiumsilikat-Glaskeramik und das Lithiumsilikatglas bis zu 70,0 Gew.-%, insbesondere 60,0 bis 70,0 Gew.-% und bevorzugt 64,0 bis 70,0 Gew.-% SiO₂.

Auch ist es bevorzugt, dass die Lithiumsilikat-Glaskeramik und das Lithiumsilikatglas 12,0 bis 17,5, insbesondere 13,0 bis 17,0 und besonders bevorzugt 14,0 bis 16,0 Gew.-% Li₂O enthalten.

In einer bevorzugten Ausführungsform beträgt das Molverhältnis von SiO₂ zu Li₂O 2,0 bis 3,0, insbesondere 2,2 bis 2,6, bevorzugt 2,3 bis 2,5 und besonders bevorzugt etwa 2,4. In einer anderen bevorzugten Ausführungsform beträgt das Molverhältnis von SiO₂ zu Li₂O weniger als 2,0, insbesondere 1,5 bis 1,9, bevorzugt 1,6 bis 1,8 und besonders bevorzugt etwa 1,7.

Es ist bevorzugt, dass die Lithiumsilikat-Glaskeramik und das Lithiumsilikatglas 5,0 bis 10,0 Gew.-%, insbesondere 5,1 bis 8,0 Gew.-%, bevorzugt 5,5 bis 7,7 Gew.-% und besonders bevorzugt 6,1 bis 7,4 Gew.-% Cs₂O enthalten.

Auch ist es bevorzugt, dass die Lithiumsilikat-Glaskeramik und das Lithiumsilikatglas 2,0 bis 6,0 Gew.-%, insbesondere 2,5 bis 5,0 und bevorzugt 3,0 bis 4,5 Gew.-% Al₂O₃ enthalten.

Vorzugsweise beträgt das Molverhältnis von Cs₂O zu Al₂O₃ mindestens 0,1, insbesondere 0,2 bis 2,0, bevorzugt 0,25 bis 1,25 und besonders bevorzugt 0,5 bis 1,0.

Vorzugsweise enthalten das Glas und die Glaskeramik als Keimbildungsmittel 1,5 bis 6,0, insbesondere 2,0 bis 4,0 und bevorzugt 2,5 bis 3,5 Gew.-% P₂O₅.

Die erfindungsgemäß verwendete Lithiumsilikat-Glaskeramik und das erfindungsgemäß verwendete Lithiumsilikatglas können darüber hinaus noch Zusatzkomponenten enthalten, die insbesondere ausgewählt sind aus weiteren Oxiden einwertiger Elemente, Oxiden zweiwertiger Elemente, weiteren Oxiden dreiwertiger Elemente, weiteren Oxiden vierwertiger Elemente, weiteren Oxiden fünfwertiger Elemente, Oxiden sechswertiger Elemente, Schmelzbeschleunigern, Färbemitteln und Fluoreszenzmitteln. In einer bevorzugten Ausführungsform enthalten die Lithiumsilikat-Glaskeramik und das Lithiumsilikatglas Zusatzkomponenten in einer Menge von 0 bis 20,0 Gew.-%, insbesondere 0,1 bis 10,0 Gew.-%, bevorzugt 0,5 bis 7,5 Gew.-% und am meisten bevorzugt 1,0 bis 5,0 Gew.-%.

Der Begriff "weitere Oxide einwertiger Elemente" bezeichnet Oxide einwertiger Elemente und insbesondere Alkalimetalloxide mit Ausnahme von Li₂O und Cs₂O. Beispiele für geeignete weitere Oxide einwertiger Elemente sind Na₂O, K₂O, Rb₂O und Mischungen davon und insbesondere Na₂O, K₂O und Mischungen davon.

In einer Ausführungsform enthalten die Lithiumsilikat-Glaskeramik und das Lithiumsilikatglas 0,1 bis 2,0 Gew.-%, insbesondere 0,2 bis 1,5 Gew.-%, bevorzugt 0,3 bis 1,4 Gew.-% und besonders bevorzugt 0,5 bis 1,0 Gew.-% Na₂O. In einer weiteren Ausführungsform enthalten die Lithiumsilikat-Glaskeramik und das Lithiumsilikatglas 0,1 bis 2,0 Gew.-%, insbesondere 0,2 bis 1,6 Gew.-%, bevorzugt 0,4 bis 1,5 Gew.-% und besonders bevorzugt 0,5 bis 1,0 Gew.-% K₂O. In einer besonders bevorzugten Ausführungsform enthalten die Lithiumsilikat-Glaskeramik und das Lithiumsilikatglas weniger als 4,0 Gew.-%, insbesondere weniger als 3,5 Gew.-%, bevorzugt weniger als 3,0 Gew.-%, besonders bevorzugt weniger als 2,5 Gew.-% und am meisten bevorzugt weniger als 2,0 Gew.-% Na₂O und/oder K₂O.

Vorzugsweise enthalten die Lithiumsilikat-Glaskeramik und das Lithiumsilikatglas weniger als 2,5 Gew.-%, insbesondere weniger als 1,5 Gew.-%, bevorzugt weniger als 1,0 Gew.-%, besonders bevorzugt weniger als 0,5 Gew.-% Rb₂O. Am meisten bevorzugt sind sie im Wesentlichen frei von Rb₂O.

Als Oxide zweiwertiger Elemente kommen insbesondere die Erdalkalimetalloxide, vorzugsweise MgO, CaO, SrO, BaO und Mischungen davon, und bevorzugt CaO, SrO und Mischungen davon in Frage.

Vorzugsweise enthalten die Lithiumsilikat-Glaskeramik und das Lithiumsilikatglas weniger als 3,8 Gew.-%, insbesondere weniger als 2,5 Gew.-%, bevorzugt weniger als 1,5 Gew.-%, besonders bevorzugt weniger als 0,5 Gew.-% BaO. Am meisten bevorzugt sind sie im Wesentlichen frei von BaO.

Der Begriff "weitere Oxide dreiwertiger Elemente" bezeichnet Oxide dreiwertiger Elemente mit Ausnahme von Al₂O₃. Geeignete Oxide dreiwertiger Elemente sind insbesondere Y₂O₃, La₂O₃, Bi₂O₃ und Mischungen davon, und bevorzugt Y₂O₃ und La₂O₃.

Der Begriff "weitere Oxide vierwertiger Elemente" bezeichnet Oxide vierwertiger Elemente mit Ausnahme von SiO₂. Beispiele für geeignete weitere Oxide vierwertiger Elemente sind TiO₂, GeO₂ und ZrO₂.

Der Begriff "weitere Oxide fünfwertiger Elemente" bezeichnet Oxide fünfwertiger Elemente mit Ausnahme von P₂O₅. Beispiele für geeignete weitere Oxide fünfwertiger Elemente sind Ta₂O₅ und Nb₂O₅.

Beispiele für geeignete Oxide sechswertiger Elemente sind WO₃ und MoO₃.

Bevorzugt sind ein Glas und eine Glaskeramik, die mindestens ein weiteres Oxid einwertiger Elemente, ein Oxid zweiwertiger Elemente, mindestens ein weiteres Oxid dreiwertiger Elemente, mindestens ein weiteres Oxid vierwertiger Elemente, mindestens ein weiteres Oxid fünfwertiger Elemente und/oder mindestens ein Oxid sechswertiger Elemente enthalten.

Beispiele für Schmelzbeschleuniger sind Fluoride.

Beispiel für Färbemittel und Fluoreszenzmittel sind Oxide von d- und f-Elementen, wie z.B. die Oxide von Ti, V, Sc, Mn, Fe, Co, Ta, W, Ce, Pr, Nd, Tb, Er, Dy, Gd, Eu und Yb. Als Färbemittel können auch Metallkolloide, z.B. von Ag, Au und Pd, verwendet werden, die zusätzlich auch als Keimbildner fungieren können. Diese Metallkolloide können z.B. durch Reduktion von entsprechenden Oxiden, Chloriden oder Nitraten während der Schmelz- und Kristallisationsprozesse gebildet werden. Die Metallkolloide sind vorzugsweise in einer Menge von 0,005 bis 0,5 Gew.-% in der Glaskeramik enthalten.

Die erfindungsgemäß verwendete Glaskeramik weist als Hauptkristallphase vorzugsweise Lithiummetasilikat und/oder Lithiumdisilikat auf. Der Begriff "Hauptkristallphase" bezeichnet dabei die Kristallphase, die gegenüber den anderen Kristallphasen den höchsten Volumenanteil hat. Wenn zwei Kristallphasen annähernd gleichen Volumenanteil haben, können diese Kristallphasen beide als Hauptkristallphasen vorliegen. In anderen Ausführungsformen kann Lithiummetasilikat als Hauptkristallphase und Lithiumdisilikat als Nebenphase oder Lithiumdisilikat als Hauptkristallphase und Lithiummetasilikat als Nebenphase vorliegen.

Es hat sich überraschenderweise gezeigt, dass die erfindungsgemäße Lithiumsilikat-Glaskeramik sehr gute mechanische und optische Eigenschaften aufweist, auch wenn bei konventionellen Glaskeramiken als wesentlich angesehene Komponenten fehlen. Die Kombination ihrer Eigenschaften erlaubt es sogar, sie als Dentalmaterial und insbesondere zur Beschichtung von Dentalrestaurationen einzusetzen.

Die erfindungsgemäße Lithiumsilikat-Glaskeramik hat vorzugsweise eine Bruchzähigkeit, gemessen als K_{IC} Wert, von mindestens etwa 2,0 MPa·m^{0,5}, insbesondere mindestens etwa 2,3 MPa·m^{0,5} und vorzugsweise mindestens etwa 2,6 MPa·m^{0,5}. Dieser Wert wurde mit dem Vicker's-Verfahren bestimmt und mittels Niihara-Gleichung berechnet. Weiter hat sie eine hohe biaxiale Bruchfestigkeit von bevorzugt 180 bis 700 MPa. Überdies zeigt sie eine hohe chemische Beständigkeit, die durch Masseverlust nach Lagerung in Essigsäure ermittelt wurde. Die chemische Beständigkeit beträgt insbesondere weniger als 100 µg/cm². Die biaxiale Bruchfestigkeit und die chemische Beständigkeit wurden gemäß ISO 6872 (2008) bestimmt.

In einer bevorzugten Ausführungsform enthält die Glaskeramik Lithiummetasilikat als Hauptkristallphase. Insbesondere enthält die Glaskeramik mehr als 5 Vol.-%, bevorzugt mehr als 10 Vol.-% und besonders bevorzugt mehr als 15 Vol.-% an Lithiummetasilikat-Kristallen, bezogen auf die gesamte Glaskeramik. Diese Lithiummetasilikat-Glaskeramik zeichnet sich durch sehr gute mechanische Eigenschaften aus. Vorzugsweise weist sie eine Biegefestigkeit im Bereich von etwa 180 bis 300 MPa und/oder eine Bruchzähigkeit, gemessen als K_{IC} Wert, von mindestens etwa 2,0 MPa·m^{0.5}, insbesondere mindestens etwa 2,3 MPa·m^{0.5} und vorzugsweise mindestens etwa 2,6 MPa·m^{0.5} auf. Sie kann z.B. durch Wärmebehandlung eines entsprechenden Lithiumsilikatglases und insbesondere eines entsprechenden Lithiumsilikatglases mit Keimen gebildet werden.

In einer weiteren besonders bevorzugten Ausführungsform enthält die Glaskeramik Lithiumdisilikat als Hauptkristallphase. Insbesondere enthält die Glaskeramik mehr als 10 Vol.-%, bevorzugt mehr als 20 Vol.-% und besonders bevorzugt mehr als 30 Vol.-% an Lithiumdisilikat-Kristallen, bezogen auf die gesamte Glaskeramik. Diese Lithiumdisilikat-Glaskeramik zeichnet sich durch besonders gute mechanische Eigenschaften aus. Vorzugsweise weist sie eine Biegefestigkeit im Bereich von etwa 400 bis 700 MPa und/oder eine Bruchzähigkeit, gemessen als K_{IC} Wert, von mindestens etwa 2,0 MPa·m^{0.5}, insbesondere mindestens etwa 2,3 MPa·m^{0.5} und vorzugsweise mindestens etwa 2,6 MPa·m^{0.5} auf. Sie kann z.B. durch Wärmebehandlung der Lithiummetasilikat-Glaskeramik erzeugt werden. Sie kann aber auch durch Wärmebehandlung eines entsprechenden Lithiumsilikatglases oder eines entsprechenden Lithiumsilikatglases mit Keimen gebildet werden.

Die Erfindung betrifft auch die Verwendung einer Lithiumsilikat-Glaskeramik, die die folgenden Komponenten enthält

| Komponente | Gew.-% | | |
|---|---|---|---|
| SiO₂ | 54,0 | bis | 78,0 |
| Li₂O | 11,0 | bis | 19,0 |
| Cs₂O | 4,0 | bis | 13,0 |
| Al₂O₃ | 1,0 | bis | 9,0 |
| P₂O₅ | 0,5 | bis | 9,0, |

zur Beschichtung eines Substrats ausgewählt aus Oxidkeramiken, Metallen und Legierungen, wobei die Lithiumsilikat-Glaskeramik mindestens eine Cäsiumaluminosilikat-Kristallphase aufweist. Es hat sich überraschend gezeigt, dass eine solche Kristallphase die Einstellbarkeit des Wärmeausdehnungskoeffizienten insbesondere zu höheren Werten verbessert. Das Cäsiumaluminosilikat hat bevorzugt die Formel CsₓAlSi₅O₁₂, wobei x 0,70 bis 0,90, insbesondere 0,75 bis 0,85, bevorzugt 0,80 bis 0,82 und besonders bevorzugt 0,808 bis 0,810 ist. Am meisten bevorzugt ist ein Cäsiumaluminosilikat, das die Formel Cs_{0,809}AlSi₅O₁₂ hat.

Die Erfindung betrifft ebenfalls die Verwendung einer Lithiumsilikat-Glaskeramik, die die folgenden Komponenten enthält

| Komponente | Gew.-% | | |
|---|---|---|---|
| SiO₂ | 54,0 | bis | 78,0 |
| Li₂O | 11,0 | bis | 19,0 |
| Cs₂O | 4,0 | bis | 13,0 |
| Al₂O₃ | 1,0 | bis | 9,0 |
| P₂O₅ | 0,5 | bis | 9,0, |

zur Beschichtung eines Substrats ausgewählt aus Oxidkeramiken, Metallen und Legierungen, wobei die Lithiumsilikat-Glaskeramik mindestens eine Lithiumaluminosilikat-Kristallphase enthält. Es hat sich überraschend gezeigt, dass eine solche Kristallphase die Einstellbarkeit des Wärmeausdehnungskoeffizienten insbesondere zu niedrigeren Werten verbessert. Das Lithiumaluminosilikat hat bevorzugt die Formel Li₂O*Al₂O₃*7,5SiO₂.

Als weitere Kristallphasen der Lithiumsilikat-Glaskeramik kommen insbesondere Li₃PO₄, SiO₂ und TiO₂ in Frage.

In einer weiteren Ausführungsform wird ein Lithiumsilikatglas verwendet. Dieses Lithiumsilikatglas enthält vorzugsweise Keime, die zur Ausbildung von Lithiummetasilikat- und/oder Lithiumdisilikatkristallen geeignet sind. Ein solches Lithiumsilikatglas mit Keimen kann insbesondere durch Wärmebehandlung eines entsprechenden Lithiumsilikatglases gebildet werden. Durch eine weitere Wärmebehandlung kann dann die erfindungsgemäße Lithiummetasilikat-Glaskeramik gebildet werden, die ihrerseits durch weitere Wärmebehandlung in die erfindungsgemäße Lithiumdisilikat-Glaskeramik umgewandelt werden kann, oder es kann auch bevorzugt direkt die erfindungsgemäße Lithiumdisilikat-Glaskeramik aus dem Glas mit Keimen gebildet werden. Mithin können das Lithiumsilikatglas, das Lithiumsilikatglas mit Keimen und die Lithiummetasilikat-Glaskeramik als Vorstufen zur Erzeugung einer erfindungsgemäßen hochfesten Lithiummeta- oder -disilikat-Glaskeramik angesehen werden. Vorzugsweise wird das Lithiumsilikatglas vor, während oder nach dem Aufbringen auf das Substrat in eine wie oben beschriebene Glaskeramik umgewandelt.

Zur Herstellung des Lithiumsilikatglases kann insbesondere so vorgegangen werden, dass eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen. Die Schmelze kann dann in Formen gegossen werden, um Rohlinge des Lithiumsilikatglases, sogenannte Massivglasrohlinge oder monolithische Rohlinge, zu erzeugen. Es ist ebenfalls möglich, die Schmelze erneut in Wasser zu geben, um ein Granulat herzustellen. Dieses Granulat kann dann nach Mahlen und gegebenenfalls Zugabe weiterer Komponenten, wie Färbe- und Fluoreszenzmitteln, zu einem Rohling, einem sogenannten Pulverpressling, gepresst werden. Schließlich kann das Lithiumsilikatglas nach Granulierung auch zu einem Pulver verarbeitet werden.

Anschließend wird das Lithiumsilikatglas, z.B. in Form eines Massivglasrohlings, eines Pulverpresslings oder in Form eines Pulvers, mindestens einer Wärmebehandlung im Bereich von 450 bis 1050 °C unterzogen. Es ist bevorzugt, dass zunächst bei einer Temperatur im Bereich von 480 bis 580 °C, insbesondere 480 bis 560 °C und bevorzugt 480 bis 520 °C eine erste Wärmebehandlung durchgeführt wird, um ein Glas mit Keimen herzustellen, welche zur Bildung von Lithiummetasilikat- und/oder Lithiumdisilikatkristallen geeignet sind. Diese erste Wärmebehandlung wird bevorzugt für eine Dauer von 5 bis 120 Minuten, insbesondere 10 bis 60 Minuten und bevorzugt 10 bis 30 Minuten durchgeführt. Das Glas mit Keimen kann dann bevorzugt mindestens einer weiteren Temperaturbehandlung bei einer höheren Temperatur und insbesondere mehr als 580 °C unterworfen werden, um Kristallisation von Lithiummetasilikat oder von Lithiumdisilikat zu bewirken. Diese weitere Wärmebehandlung wird bevorzugt für eine Dauer von 10 bis 120 Minuten, insbesondere 10 bis 60 Minuten und besonders bevorzugt 20 bis 30 Minuten durchgeführt. Zur Kristallisation von Lithiummetasilikat erfolgt die weitere Wärmebehandlung üblicherweise bei 600 bis 950 °C, bevorzugt 620 bis 850 °C und ganz besonders bevorzugt 650 bis 750 °C. Zur Kristallisation von Lithiumdisilikat erfolgt die weitere Wärmebehandlung üblicherweise bei 750 bis 1050 °C, bevorzugt 800 bis 1000 °C, besonders bevorzugt 820 bis 950 °C und ganz besonders bevorzugt 850 bis 900 °C.

Die erfindungsgemäß verwendete Lithiumsilikat-Glaskeramik und das erfindungsgemäß verwendete Lithiumsilikatglas liegen insbesondere in Form von Pulvern, Granulaten oder Rohlingen, z.B. monolithische Rohlingen, wie Plättchen, Quadern oder Zylinder, oder Pulverpresslingen, in ungesinterter, teilgesinterter oder dichtgesinterter Form, vor. In diesen Formen können sie einfach weiterverarbeitet werden. Sie können aber auch in Form eines Überwurfs für dentale Restaurationen, wie insbesondere Kronen, vorliegen. Dabei ist es bevorzugt, dass die Glaskeramik oder das Glas durch maschinelle Bearbeitung oder Verpressen zur gewünschten Geometrie verformt werden.

Die erfindungsgemäß verwendete Lithiumsilikat-Glaskeramik und das erfindungsgemäß verwendete Lithiumsilikatglas eignen sich insbesondere zur Beschichtung von Oxidkeramiken, Metallen und Legierungen.

In einer bevorzugten Ausführungsform ist das Substrat eine Oxidkeramik. Dabei sind Zirkonoxidkeramiken besonders bevorzugt. Beispiele für geeignete Zirkonoxidkeramiken sind Keramiken auf Basis von polykristallinem tetragonalen Zirkoniumoxid (tetragonal zirconia polycrystal, TZP), bei denen die tetragonale Form durch Zusatz von Y₂O₃ und/oder CeO₂ stabilisiert ist.

Die Erfindung betrifft auch die Verwendung einer Lithiumsilikat-Glaskeramik oder eines Lithiumsilikatglases, die die folgenden Komponenten enthalten

| Komponente | Gew.-% | | |
|---|---|---|---|
| SiO₂ | 54,0 | bis | 78,0 |
| Li₂O | 11,0 | bis | 19,0 |
| Cs₂O | 4,0 | bis | 13,0 |
| Al₂O₃ | 1,0 | bis | 9,0 |
| P₂O₅ | 0,5 | bis | 9,0, |

zur Beschichtung eines Substrats, wobei das Substrat eine Nichtedelmetall-Legierung ist. Dabei sind Nichtedelmetall-Legierungen und insbesondere Nichteisen-Legierungen, die für dentale Anwendungen geeignet sind, besonders bevorzugt. Beispiele für geeignete Legierungen sind insbesondere Legierungen vom Typ Ni-Cr, Co-Cr und Co-Cr-W.

Weiterhin ist es bevorzugt, dass das Substrat eine Dentalrestauration und insbesondere eine Brücke, ein Inlay, ein Onlay, ein Veneer, ein Abutment, eine Teilkrone, eine Krone oder eine Schale ist.

Die Erfindung betrifft auch ein Verfahren zur Beschichtung eines Substrats ausgewählt aus Metallen und Legierungen, bei dem eine wie oben beschriebene Lithiumsilikat-Glaskeramik oder ein wie oben beschriebenes Lithiumsilikatglas auf das Substrat aufgebracht werden. Vorzugsweise handelt es sich dabei um ein wie vorstehend beschriebenes Substrat.

In einer Ausführungsform des erfindungsgemäßen Verfahrens werden die Lithiumsilikat-Glaskeramik oder das Lithiumsilikatglas durch Aufsintern und bevorzugt durch Aufpressen auf das Substrat aufgebracht.

Beim Aufsintern werden die erfindungsgemäße Lithiumsilikat-Glaskeramik oder das erfindungsgemäße Lithiumsilikatglas in üblicher Weise, z.B. als Pulver, auf das zu beschichtende Material aufgebracht und anschließend bei erhöhter Temperatur gesintert.

Bei dem bevorzugten Aufpressen werden die erfindungsgemäße Lithiumsilikat-Glaskeramik oder das erfindungsgemäße Lithiumsilikatglas, z.B. in Form von Pulverpresslingen oder monolithischen Rohlingen, bei einer erhöhten Temperatur, von z.B. 700 bis 1200°C, in einen viskosen Zustand überführt und unter Anwendung von geringem Druck, z.B. 2 bis 10 bar, auf das Substrat aufgepresst. Hierzu können insbesondere die in der EP 231 773 A1 beschriebenen Verfahren und der dort offenbarte Pressofen eingesetzt werden. Ein geeigneter Ofen ist z.B. der Programat EP 5000 der Ivoclar Vivadent AG.

In einer anderen Ausführungsform des erfindungsgemäßen Verfahrens werden die Lithiumsilikat-Glaskeramik oder das Lithiumsilikatglas durch Fügen auf das Substrat aufgebracht. Geeignete Fügeverfahren sind an sich bekannt und umfassen beispielsweise Fügen mittels eines Glas- oder Glaskeramiklots, Fügen durch Kleben mittels eines Adhäsivs oder Dentalzements, Fügen durch Senken mittels einer Temperaturbehandlung, bei der die zu fügenden Werkstoffe erweicht werden, und Fügen durch Reibverschweißung oder Ansprengen.

In einer besonders bevorzugten Ausführungsform werden die Lithiumsilikat-Glaskeramik oder das Lithiumsilikatglas vor dem Fügen durch Heißpressen oder durch maschinelle Bearbeitung zu einer gewünschten Geometrie verformt.

Das Heißpressen erfolgt üblicherweise unter erhöhtem Druck und erhöhter Temperatur. Es ist bevorzugt, dass das Heißpressen bei einer Temperatur von 700 bis 1200°C erfolgt. Weiter ist es bevorzugt, das Heißpressen bei einem Druck von 2 bis 10 bar durchzuführen. Dabei wird durch viskoses Fließen des eingesetzten Materials die gewünschte Formänderung erreicht. Für das Heißpressen können die erfindungsgemäße Lithiummetasilikat-Glaskeramik, die erfindungsgemäße Lithiumdisilikat-Glaskeramik, das erfindungsgemäße Lithiumsilikatglas und insbesondere das erfindungsgemäße Lithiumsilikatglas mit Keimen verwendet werden. Dabei können die Glaskeramiken und Gläser insbesondere in Form von Rohlingen, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das Lithiumsilikatglas, das Lithiumsilikatglas mit Keimen, die Lithiummetasilikat- und die Lithiumdisilikat-Glaskeramik verwendet werden. Dabei können die Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt Lithiumsilikat-Glaskeramik insbesondere mit Lithiumdisilikat und bevorzugt mit Lithiummetasilikat als Hauptkristallphase verwendet. Die Lithiumsilikat-Glaskeramik kann auch in einer noch nicht vollständig kristallisierten Form eingesetzt werden, die durch Wärmebehandlung bei niedrigerer Temperatur erzeugt wurde. Dies bietet den Vorteil, dass eine leichtere maschinelle Bearbeitung und damit der Einsatz von einfacheren Apparaten zur maschinellen Bearbeitung möglich ist. Nach der maschinellen Bearbeitung eines solchen teilkristallisierten Materials wird dieses regelmäßig einer Wärmebehandlung bei höherer Temperatur und insbesondere 750 bis 1050 °C, bevorzugt 800 bis 950 °C und besonders bevorzugt etwa 850 bis 900 °C unterzogen, um weitere Kristallisation von Lithiummetasilikat oder vorzugsweise Lithiumdisilikat hervorzurufen.

Allgemein können die Lithiumsilikat-Glaskeramik oder das Lithiumsilikatglas nach der Verformung durch Heißpressen oder maschinelle Bearbeitung insbesondere noch wärmebehandelt werden, um eingesetzte Vorläufer, wie Lithiumsilikatglas, Lithiumsilikatglas mit Keimen oder Lithiummetasilikat-Glaskeramik, in Lithiummeta- und/oder - disilikat-Glaskeramik umzuwandeln, die Kristallisation von Lithiummeta- und/oder -disilikat zu steigern, oder die Porosität, z.B. eines eingesetzten porösen Pulverpresslings, zu vermindern.

Es ist bevorzugt, dass nach Abschluss des Beschichtungsvorganges eine Beschichtung erhalten wird, die eine Lithiumsilikat-Glaskeramik mit Lithiummeta- und/oder -disilikat als Hauptkristallphase umfasst, da sie über besonders gute Eigenschaften verfügt. Dabei sind Glaskeramiken besonders bevorzugt, die die oben beschriebenen Kristallphasen und mechanischen Eigenschaften aufweisen.

Weiterhin betrifft die Erfindung einen Verbundwerkstoff, der eine wie oben definierte Lithiumsilikat-Glaskeramik oder ein wie oben definiertes Lithiumsilikatglas auf einem Substrat ausgewählt aus Metallen und Legierungen umfasst. Dabei sind alle Ausführungsformen bevorzugt, die auch für die erfindungsgemäß verwendete Lithiumsilikat-Glaskeramik, das erfindungsgemäß verwendete Lithiumsilikatglas sowie das Substrat als bevorzugt angegeben sind. Der Verbundwerkstoff kann insbesondere mittels des erfindungsgemäßen Verfahrens hergestellt werden.

Die Erfindung betrifft auch eine Lithiumsilikat-Glaskeramik, die die folgenden Komponenten enthält

| Komponente | Gew.-% | | | | | | |
|---|---|---|---|---|---|---|---|
| SiO₂ | 54,0 | bis | 78,0, | insbesondere | 55,1 | bis | 77,1 |
| Li₂O | 11,8 | bis | 19,0, | insbesondere | 11,8 | bis | 17,8 |
| Cs₂O | 4,6 | bis | 13,0, | insbesondere | 4,7 | bis | 11,7 |
| Al₂O₃ | 1,7 | bis | 9,0, | insbesondere | 1,7 | bis | 8,0 |
| P₂O₅ | 0,5 | bis | 9,0, | insbesondere | 1,3 | bis | 7,5 |
| Rb₂O | 0 | bis | 7,0, | insbesondere | 0 | bis | 5,0 |
| BaO | 0 | bis | 3,7, | insbesondere | 0 | bis | 3,0 |
| ZrO₂ | 0 | bis | 4,5, | insbesondere | 0 | bis | 4,0 |
| Übergangsmetalloxid | 0 | bis | 7,5, | insbesondere | 0 | bis | 7,0, |

wobei das Übergangsmetalloxid ausgewählt ist aus der Gruppe bestehend aus Oxiden von Yttrium, Oxiden von Übergangsmetallen mit Ordnungszahl 41 bis 79 und Mischungen dieser Oxide.

Außerdem betrifft die Erfindung auch ein Lithiumsilikatglas, das die Komponenten der vorstehenden Glaskeramik enthält.

Das Lithiumsilikatglas und die Lithiumsilikat-Glaskeramik können darüber hinaus auch noch andere Komponenten enthalten, wie sie oben für die erfindungsgemäß verwendete Lithiumsilikat-Glaskeramik und das erfindungsgemäß verwendete Lithiumsilikatglas angegeben sind. Dabei sind alle Ausführungsformen bevorzugt, die auch für die erfindungsgemäß verwendete Lithiumsilikat-Glaskeramik und das erfindungsgemäß verwendete Lithiumsilikatglas als bevorzugt angegeben sind.

Die Erfindung wird im Folgenden anhand von Beispielen näher erläutert.

### Beispiele

Es wurden insgesamt 28 erfindungsgemäße Gläser und Glaskeramiken mit den in Tabelle I angegebenen Zusammensetzungen über Erschmelzung entsprechender Ausgangsgläser und anschließende Wärmebehandlung zur gesteuerten Keimbildung und Kristallisation hergestellt.

Dazu wurden zunächst die Ausgangsgläser im 100 bis 200 g Maßstab aus üblichen Rohstoffen bei 1450 bis 1550 °C erschmolzen, wobei das Erschmelzen sehr gut ohne Bildung von Blasen oder Schlieren möglich war. Durch Eingießen der Ausgangsgläser in Wasser wurden Glasfritten hergestellt, die zur Homogenisierung anschließend ein zweites Mal bei 1450 bis 1550 °C für 1 bis 3 h geschmolzen wurden. Die erhaltenen Glasschmelzen wurden dann in vorgewärmte Formen gegossen, um Glasmonolithe zu erzeugen. Alle Glasmonolithe erwiesen sich als transparent.

Die Glasmonolithe wurden dann durch thermische Behandlung zu erfindungsgemäßen Gläsern und Glaskeramiken umgewandelt. Die angewendeten thermischen Behandlungen zur gesteuerten Keimbildung und gesteuerten Kristallisation sind ebenfalls in Tabelle I angegeben. Dabei bedeuten

| | |
|---|---|
| T_{N} und t_{N} | Angewendete Temperatur und Zeit für Keimbildung |
| T_{C} und t_{C} | Angewendete Temperatur und Zeit für erste Kristallisation |
| T_{FC} und t_{FC} | Angewendete Temperatur und Zeit für endgültige Kristallisation |
| Tₚᵣₑₛₛ und tₚᵣₑₛₛ | Angewendete Temperatur und Zeit für Heißpressen |

Es ist ersichtlich, dass eine erste Wärmebehandlung im Bereich von 470 bis 580 °C zur Bildung von Lithiumsilikat-Gläsern mit Keimen führte und diese Gläser durch eine weitere Wärmebehandlung bei 600 bis 750 °C zu Glaskeramiken mit Lithiummetasilikat als Hauptkristallphase kristallisierten, wie durch Röntgenbeugungsuntersuchungen festgestellt wurde. Lediglich im Falle der Beispiele 8, 11 und 12 wurde (auch) Lithiumdisilikat als Hauptkristallphase gebildet. Eine abschließende Wärmebehandlung bei einer Temperatur von 840 bis 950 °C führte schließlich überwiegend zur Bildung von Glaskeramiken mit Lithiumdisilikat als Hauptkristallphase. Im Falle der Beispiele 3, 5 und 9 wurden Glaskeramiken mit Lithiummetasilikat als Hauptkristallphase erhalten.

Die erzeugten Lithiumdisilikat-Glaskeramiken hatten hohe Bruchzähigkeiten, gemessen als kritischer Spannungsintensitätsfaktor K_{IC} nach der SEVNB-Methode, von mehr als 2,0 MPa•m^{0,5} und insbesondere sogar mindestens 2,6 MPa•m^{0,5}.

Auch die Biaxialfestigkeit σ_{B} war mit mindestens 250 MPa und bis zu mehr als 600 MPa hoch. Sie wurde gemäß Dentalnorm ISO 6872 (2008) an Prüfkörpern bestimmt, die durch maschinelle Bearbeitung der jeweiligen Lithiumdisilikat-Glaskeramik hergestellt wurden. Zur Bearbeitung wurde eine CEREC-InLab Maschine (Sirona, Bensheim) verwendet.

Ebenfalls konnten sie durch Heißpressen als Beschichtungen insbesondere auf Oxidkeramikrestaurationen oder Metallrestaurationen aufgebracht werden, z.B. um diese in gewünschter Weise zu verblenden.

Im Folgenden werden einige Beispiele näher beschrieben:

### Beispiel 10

Aus einer Mischung der Rohstoffe mit der in Tabelle I für Beispiel 10 angegebenen Zusammensetzung wurde bei einer Temperatur von 1500 °C für 2 h ein Glas erschmolzen und durch anschließendes Eingießen in Wasser eine Glasfritte hergestellt. Nach dem Trocknen der Glasfritte im Trockenschrank bis 750 °C wurde diese erneut bei 1500 °C für 2,5 h aufgeschmolzen und dann in Graphit-Formen gegossen, um Glasmonolithe herzustellen. Unmittelbar nach der Entformung der heißen Glasmonolithe wurden diese für 10 min bei 500 °C entspannt und keimgebildet und anschließend langsam auf Raumtemperatur abgekühlt.

Vor der weiteren Bearbeitung wurden die Glasblöcke in eine Lithiummetasilikat-Glaskeramik umgewandelt. Dazu wurden die Glasblöcke in einen auf 400°C vorgewärmten Ofen gegeben. Nach einer Haltezeit von 20 min wurde die Temperatur mit einer Aufheizrate von 10 K/min auf 700 °C erhöht und diese Temperatur für 20 min gehalten. Im geschlossenen Ofen wurden die Blöcke anschließend langsam auf Raumtemperatur abgekühlt.

Um eine CAM-Bearbeitung der Metasilikatblöcke mittels Sirona inLab Schleifmaschinen zu ermöglichen, wurden die entsprechenden Halter auf die Blöcke aufgeklebt. Die schleifende Bearbeitung erfolgte mit diamantbeschichteten Schleifwerkzeugen. Aus den Blöcken wurden Plättchen mit einem Durchmesser von ca. 12 mm und einer Dicke von ca. 2 mm herausgeschliffen.

Die Überführung der geschliffenen Körper aus dem Metasilikatzustand in den Disilikatzustand erfolgte über eine weitere thermische Behandlung. Dabei wurden die Körper in einem Ofen vom Typ Programat (Ivoclar Vivadent AG) auf eine Temperatur von 920 °C erhitzt und nach einer Haltezeit von 7 min langsam auf eine Temperatur von 400°C abgekühlt und aus dem Ofen genommen. Die XRD-Analyse des so hergestellten Materials zeigte neben der Hauptphase Li₂Si₂O₅ unter anderem die Nebenphase Cs_{0.809} (AlSi₅O₁₂), welche für einen hohen thermischen Ausdehnungskoeffizienten der Glaskeramiken verantwortlich ist. Die so hergestellte Glaskeramik eignet sich dadurch zum Fügen auf Metallen und Metalllegierungen. Die kristallisierten Plättchen wurden daraufhin mit Diamantschleifscheiben auf eine Dicke von ca. 1.2 mm geschliffen und bis 0,5 µm poliert. An den so hergestellten und präparierten Proben wurde daraufhin die Biaxialfestigkeit bestimmt. Es wurde eine mittlere Festigkeit von 608 MPa gemessen.

### Beispiel 14

Aus einer Mischung der Rohstoffe mit der in Tabelle I für Beispiel 14 angegebenen Zusammensetzung wurde bei einer Temperatur von 1500°C für 2 h ein Glas erschmolzen und durch anschließendes Eingießen in Wasser eine Glasfritte hergestellt. Die getrocknete Glasfritte wurde daraufhin erneut bei 1500 °C für 2,5 h geschmolzen und dann zur Herstellung von Glasmonolithen in Graphit-Formen gegossen. Die Entspannung und Keimbildung der so hergestellten Glasblöcke erfolgte unmittelbar nach der Entformung bei 500 °C für 10 min, gefolgt von einer langsamen Abkühlung auf Raumtemperatur.

Vor der weiteren Bearbeitung wurden die Glasblöcke in den Metasilikatzustand überführt. Dazu wurden die Glasblöcke in einen auf 400°C vorgewärmten Ofen gegeben. Nach einer Haltezeit von 20 min wurde die Temperatur mit einer Aufheizrate von 10 K/min auf 600 °C erhöht und diese Temperatur für 120 min gehalten. Im geschlossenen Ofen wurden die Blöcke anschließend langsam auf Raumtemperatur abgekühlt. An den so kristallisierten Blöcken wurde eine Röntgenstrukturanalyse durchgeführt. Li₂SiO₃ konnte als Hauptkristallphase identifiziert werden.

Um eine CAM-Bearbeitung der Metasilikatblöcke mittels Sirona inLab Schleifmaschinen zu ermöglichen, wurden die entsprechenden Halter auf die Blöcke aufgeklebt. Die schleifende Bearbeitung erfolgte mit diamantbeschichteten Schleifwerkzeugen. Aus den Blöcken wurden Plättchen mit einem Durchmesser von ca. 12 mm und einer Dicke von ca. 2 mm herausgeschliffen.

Die Überführung der geschliffenen Körper aus dem Metasilikatzustand in den Disilikatzustand erfolgte über eine weitere thermische Behandlung. Dabei wurden die Körper in einem Ofen vom Typ Programat (Ivoclar Vivadent AG) auf eine Temperatur von 905 °C erhitzt und nach einer Haltezeit von 30 min langsam auf eine Temperatur von 400 °C abgekühlt und aus dem Ofen genommen. Eine XRD-Analyse des so behandelten Materials zeigte neben der Hauptkristallphase Li₂Si₂O₅ unter anderem eine Lithiumaluminosilikat-Nebenphase. Der lineare Wärmeausdehnungskoeffizient WAK_{100-400°C} betrug 9,0·10⁻⁶K⁻¹. Aufgrund des niedrigen thermischen Ausdehnungskoeffizienten ist die so hergestellte Glaskeramik geeignet zum Fügen auf Zirkonoxid-Keramiken.

### Beispiel 15

Aus einer Mischung der Rohstoffe mit der in Tabelle I für Beispiel 15 angegebenen Zusammensetzung wurde bei einer Temperatur von 1500 °C für 2 h ein Glas erschmolzen und durch anschließendes Eingießen in Wasser eine Glasfritte hergestellt. Die getrocknete Glasfritte wurde daraufhin erneut bei 1500 °C für 2,5 h geschmolzen und dann zur Herstellung von Glasmonolithen in Graphit-Formen gegossen. Die Entspannung und Keimbildung der so hergestellten Glasblöcke erfolgte unmittelbar nach der Entformung bei 500 °C für 10 min, gefolgt von einer langsamen Abkühlung auf Raumtemperatur.

### a) CAM-Bearbeitung von Metasilikatblöcken und Überführung in Lithiumdisilikat

Zur Überführung in den Metasilikatzustand wurden die Glasblöcke in einen auf 400 °C vorgewärmten Ofen gegeben. Nach einer Haltezeit von 20 min wurde die Temperatur mit einer Aufheizrate von 10 K/min auf 650 °C erhöht und diese Temperatur für 60 min gehalten. Im geschlossenen Ofen wurden die Blöcke anschließend langsam auf Raumtemperatur abgekühlt. An den so kristallisierten Blöcken wurde eine Röntgenstrukturanalyse durchgeführt. Li₂SiO₃ konnte als Hauptkristallphase identifiziert werden.

Um eine CAM-Bearbeitung der Metasilikatblöcke mittels Sirona inLab Schleifmaschinen zu ermöglichen, wurden die entsprechenden Halter auf die Blöcke aufgeklebt. Die schleifende Bearbeitung erfolgte mit diamantbeschichteten Schleifwerkzeugen. Aus den Blöcken wurden Plättchen mit einem Durchmesser von ca. 12 mm und einer Dicke von ca. 2 mm herausgeschliffen.

Die Überführung der geschliffenen Körper aus dem Metasilikatzustand in den Disilikatzustand erfolgte über eine weitere thermische Behandlung. Dabei wurden die Körper in einem Ofen vom Typ Programat (Ivoclar Vivadent AG) auf eine Temperatur von 920 °C erhitzt und nach einer Haltezeit von 60 min langsam auf eine Temperatur von 400 °C abgekühlt und aus dem Ofen genommen. Die XRD-Analyse des so hergestellten Materials zeigte neben der Hauptphase Li₂Si₂O₅ unter anderem die Nebenphase Cs_{0.809} (AlSi₅O₁₂). Der lineare Wärmeausdehnungskoeffizient WAK_{100-400°C} betrug 13,1·10⁻⁶ K⁻¹. Die so hergestellte Glaskeramik eignet sich dadurch zum Fügen auf Metallen und Metalllegierungen.

Die kristallisierten Plättchen wurden daraufhin mit Diamantschleifscheiben auf eine Dicke von ca. 1.2 mm geschliffen und bis 0,5 µm poliert. An den so hergestellten und präparierten Proben wurde daraufhin eine Biaxialfestigkeit bestimmt. Es wurde eine mittlere Festigkeit von 350 MPa gemessen.

### b) CAM-Bearbeitung von Metasilikatblöcken, Überführung in Lithiumdisilikat und Fügen auf Legierung

Ein weiterer Glasblock wurde über eine Temperaturbehandlung bei 600 °C für 120 min in eine Glaskeramik mit der Hauptkristallphase Li₂SiO₃ überführt. Der Metasilikatblock wurde daraufhin mit einem Halter passend für die Bearbeitung durch Sirona inLab-Schleifmaschinen versehen und es wurde aus dem Block ein Überwurf herausgeschliffen. Der Überwurf wurde daraufhin durch eine Temperaturbehandlung bei 940 °C für 15 min in den Disilikatzustand überführt. Eine Gerüststruktur aus der Legierung d.SIGN® 30 (Ivoclar Vivadent AG) wurde mit dem Opaker IPS Classic (Ivoclar Vivadent AG) opakiert und anschließend gebrannt. Unter Zuhilfenahme eines silikatischen Glaskeramiklotes mit einem thermischen Ausdehnungskoeffizienten von ca. 13,5·10⁻⁶ K⁻¹ wurde daraufhin das Gerüst mit dem Überwurf bei einer Fügetemperatur von 800 °C für 7 min gefügt.

### Beispiel 22

Aus einer Mischung der Rohstoffe mit der in Tabelle I für Beispiel 22 angegebenen Zusammensetzung wurde bei einer Temperatur von 1500 °C für 2 h ein Glas erschmolzen und durch anschließendes Eingießen in Wasser eine Glasfritte hergestellt. Nach dem Trocknen der Glasfritte im Trockenschrank wurde diese erneut bei 1500 °C für 2 h aufgeschmolzen und dann in Graphit-Formen gegossen, um Glasmonolithe herzustellen. Unmittelbar nach der Entformung der heißen Glasmonolithe wurden diese für 10 min bei 480 °C entspannt und keimgebildet und anschließend langsam auf Raumtemperatur abgekühlt.

### a) CAM-Bearbeitung von Metasilikatblöcken und Überführung in Lithiumdisilikat

Um eine CAM-Bearbeitung der Metasilikatblöcke mittels Sirona inLab Schleifmaschinen zu ermöglichen, wurden die entsprechenden Halter auf die Blöcke aufgeklebt. Die schleifende Bearbeitung erfolgte mit diamantbeschichteten Schleifwerkzeugen. Aus den Blöcken wurden Plättchen mit einem Durchmesser von ca. 12 mm und einer Dicke von ca. 2 mm herausgeschliffen.

Die Überführung der geschliffenen Körper in den Disilikatzustand erfolgte über eine thermische Behandlung. Dabei wurden die Körper in einem Ofen vom Typ Programat (Ivoclar Vivadent AG) auf eine Temperatur von 900°C erhitzt und nach einer Haltezeit von 7 min langsam auf eine Temperatur von 400 °C abgekühlt und aus dem Ofen genommen.

Die kristallisierten Plättchen wurden daraufhin mit Diamantschleifscheiben auf eine Dicke von ca. 1.2 mm geschliffen und bis 0,5 µm poliert. An den so hergestellten und präparierten Proben wurde daraufhin die Biaxialfestigkeit bestimmt. Es wurde eine mittlere Festigkeit von 319 MPa gemessen.

### b) Heißpressen von keimgebildetem Glas zu Restaurationen

Für das Heißpressen wurden Restaurationen verschiedener Geometrien aus Kunststoff (PMMA) in dentaler Einbettmasse eingebettet, der Kunststoff wurde ausgebrannt und die keimgebildeten Glasblöcke wurden bei einer Temperatur von 950 °C (Haltezeit 25 min) und Verwendung eines EP5000 innerhalb von 2,29 min verpresst. Nach dem vollständigen Abkühlen wurden die Restaurationen mit einem Sandstrahlgerät ausgebettet und von ihren Presskanälen abgetrennt und beschliffen. Der lineare Wärmeausdehnungskoeffizient WAK_{100-400°C} betrug 11,9·10⁻⁶ K⁻¹.

### Beispiel 23A

Eine Mischung von Oxiden und Carbonaten mit der in Tabelle I für Beispiel 23 angegebenen Zusammensetzung wurde zunächst in einem Speed Mixer für 2 Minuten gemischt und anschließend bei 1500 °C für 2 h in einem Platin/Rhodium-Tiegel (Pt/Rh-90/10) eingeschmolzen. Die Schmelze wurde dann in Wasser gegossen, um ein feinteiliges Granulat zu erhalten, welches nach dem Trocknen erneut 2 h bei 1500 °C zur Verbesserung der Homogenität geschmolzen wurde. Anschließend wurden zylindrische Glasrohlinge mit einem Durchmesser von 12,5 mm in vorgeheizte, teilbare Stahlformen gegossen, in einen auf 470 °C vorgeheizten Ofen überführt und für 10 min entspannt. Auf diese Weise wurde ein Glas mit Keimen erhalten.

### a) Heißpressen von keimgebildetem Glas zu Restaurationen

Für das Heißpressen wurden Restaurationen verschiedener Geometrien aus Kunststoff (PMMA) in dentaler Einbettmasse eingebettet, der Kunststoff wurde ausgebrannt und die keimgebildeten Glaszylinder wurden bei einer Temperatur von 920 °C (Haltezeit 25 min) und Verwendung eines EP5000 innerhalb von 4,20 min verpresst. Nach dem vollständigen Abkühlen wurden die Restaurationen mit einem Sandstrahlgerät ausgebettet und von ihren Presskanälen abgetrennt und beschliffen. Der lineare Wärmeausdehnungskoeffizient WAK_{100-400°C} betrug 12,1·10⁻⁶ K⁻¹.

### b) Heißpressen von keimgebildetem Glas auf Legierung

Das Glas mit Keimen wurde 30 min bei 890 °C kristallisiert und durch Heißpressen bei 940 °C auf eine Metallegierung d.Sign 30 (Ivoclar Vivadent AG) in einem Ofen vom Typ Programat EP 5000 (Ivoclar Vivadent AG) aufgepresst. Als Zwischenschicht zwischen Metall und Pressglaskeramik wurde ein ZrO₂-haltiger Opaker aus dem Sortiment IPS classic® (Ivoclar Vivadent AG) verwendet. Nach dem Pressvorgang konnte eine defektfreie Oberfläche erreicht werden. Somit ist diese Glaskeramik zum Überpressen von Dentalkronen aus Nichtedelmetall-Legierungen, insbesondere von Co-Cr und Ni-Cr-Legierungen, geeignet.

### Beispiel 23B

Aus den Rohstoffen des Beispiels 23 wurde bei Temperaturen zwischen 1450 und 1550 °C für 1 bis 3 h ein Glas erschmolzen und durch anschließendes Eingießen in Wasser eine Glasfritte hergestellt. Nach dem Trocknen der Glasfritte im Trockenschrank wurde diese auf eine Korngröße von < 45 µm zerkleinert.

### a) Schichten von Glaspulver auf Legierung und Sintern

Das Glaspulver wurde mit einer Modellierflüssigkeit gemischt und die Mischung wurde auf eine Einzelzahnkrone der Legierung IPS d.SIGN® 30 (Ivoclar Vivadent AG) aufgebracht. Als Zwischenschicht zwischen Metall und geschichtetem Pulver wurde der Opaker aus dem Sortiment IPS classic® (Ivoclar Vivadent AG) verwendet. Im Anschluss daran wurde die aufgeschichtete Mischung in einem Dentalofen P100 (Ivoclar Vivadent AG) bei einer Haltetemperatur von 850 °C und einer Haltezeit von 5 min zweimalig aufgesintert (2 Korrekturbrände). Nach dem Aufsintern folgten das Auftragen der Universalglasur (Ivoclar Vivadent AG) und ein Glasurbrand bei 850 °C und einer Haltezeit von 5 min.

### b) Schichten eines gesinterten Glases auf Legierung und Sintern

Das Glaspulver wurde zu Rohlingen mit einem Gewicht von 40 g verarbeitet und für eine Stunde bei 940 °C gesintert. Im Anschluss daran wurde der gesinterte Körper 1,30 min an der Luft gekühlt und anschließend in Wasser abgeschreckt und getrocknet. Dieser gesinterte Körper wurde erneut auf eine Korngröße von < 45 µm zerkleinert und analog dem Glaspulver auf eine Einzelzahnkrone aus der Metalllegierung IPS d.SIGN® 30 aufgeschichtet. Als Zwischenschicht zwischen Metall und geschichtetem Pulver wurde ebenfalls der Opaker aus dem Sortiment IPS classic® (Ivoclar Vivadent AG) verwendet. Die Korrekturbrände erfolgten bei 900 °C für 5 min.

### Beispiel 26

Eine Mischung von Oxiden und Carbonaten mit der in Tabelle I für Beispiel 26 angegebenen Zusammensetzung wurde zunächst in einem Speed Mixer für 2 Minuten gemischt und anschließend bei 1500 °C für 2 h in einem Platin/Rhodium-Tiegel (Pt/Rh-90/10) eingeschmolzen. Die Schmelze wurde dann in Wasser gegossen, um ein feinteiliges Granulat zu erhalten, welches nach dem Trocknen erneut 2 h bei 1500°C zur Verbesserung der Homogenität geschmolzen wurde. Anschließend wurden zylindrische Glasrohlinge mit einem Durchmesser von 12,5 mm in vorgeheizte, teilbare Stahlformen gegossen und in einen auf 480 °C vorgeheizten Ofen überführt und für 10 Minuten entspannt. Auf diese Weise wurde ein Glas mit Keimen erhalten.

### a) Heißpressen von keimgebildetem Glas zu Restaurationen

Für das Heißpressen wurden Restaurationen verschiedener Geometrien aus Kunststoff (PMMA) in dentaler Einbettmasse eingebettet, der Kunststoff wurde ausgebrannt und die keimgebildeten Glaszylinder wurden bei einer Temperatur von 930 °C (Haltezeit 25 min) und Verwendung eines EP5000 innerhalb von 1,25 min verpresst. Nach dem vollständigen Abkühlen wurden die Restaurationen mit einem Sandstrahlgerät ausgebettet und von ihren Presskanälen abgetrennt und beschliffen. Der lineare Wärmeausdehnungskoeffizient WAK_{100-400°C} betrug 12,1·10⁻⁶ K⁻¹.

### b) Heißpressen auf Zirkonoxidkeramik

Das Glas wurde durch Heißpressen bei 940 °C auf eine Zirkonoxidkeramik des Typs 3Y-TZP (Fa. Tosoh, Japan) in einem Pressofen Programat EP5000 (Ivoclar Vivadent AG) durchgeführt. Nach Abschluss des Beschichtungsprozesses konnte eine defektfreie Glaskeramik-Krone erhalten werden.

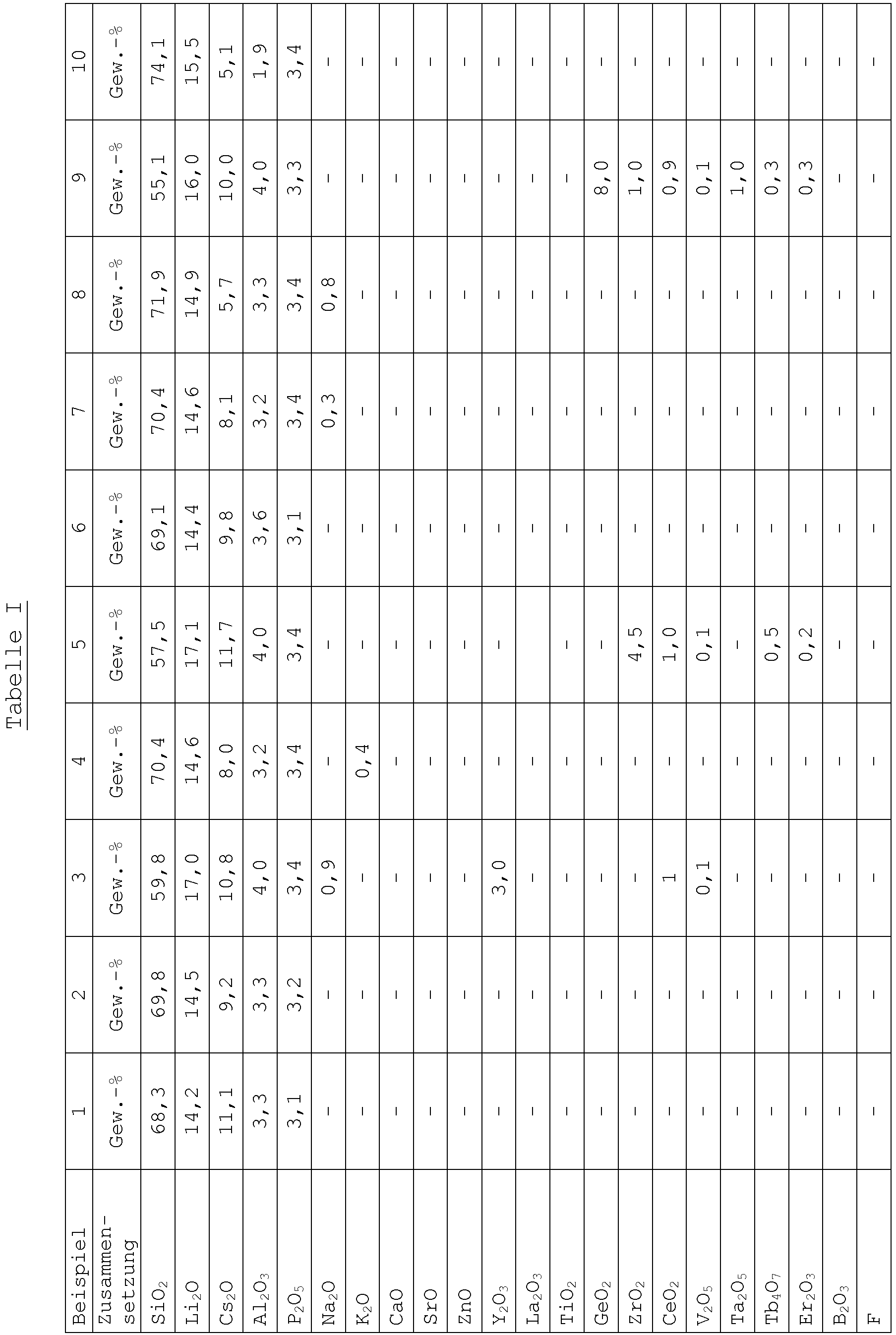

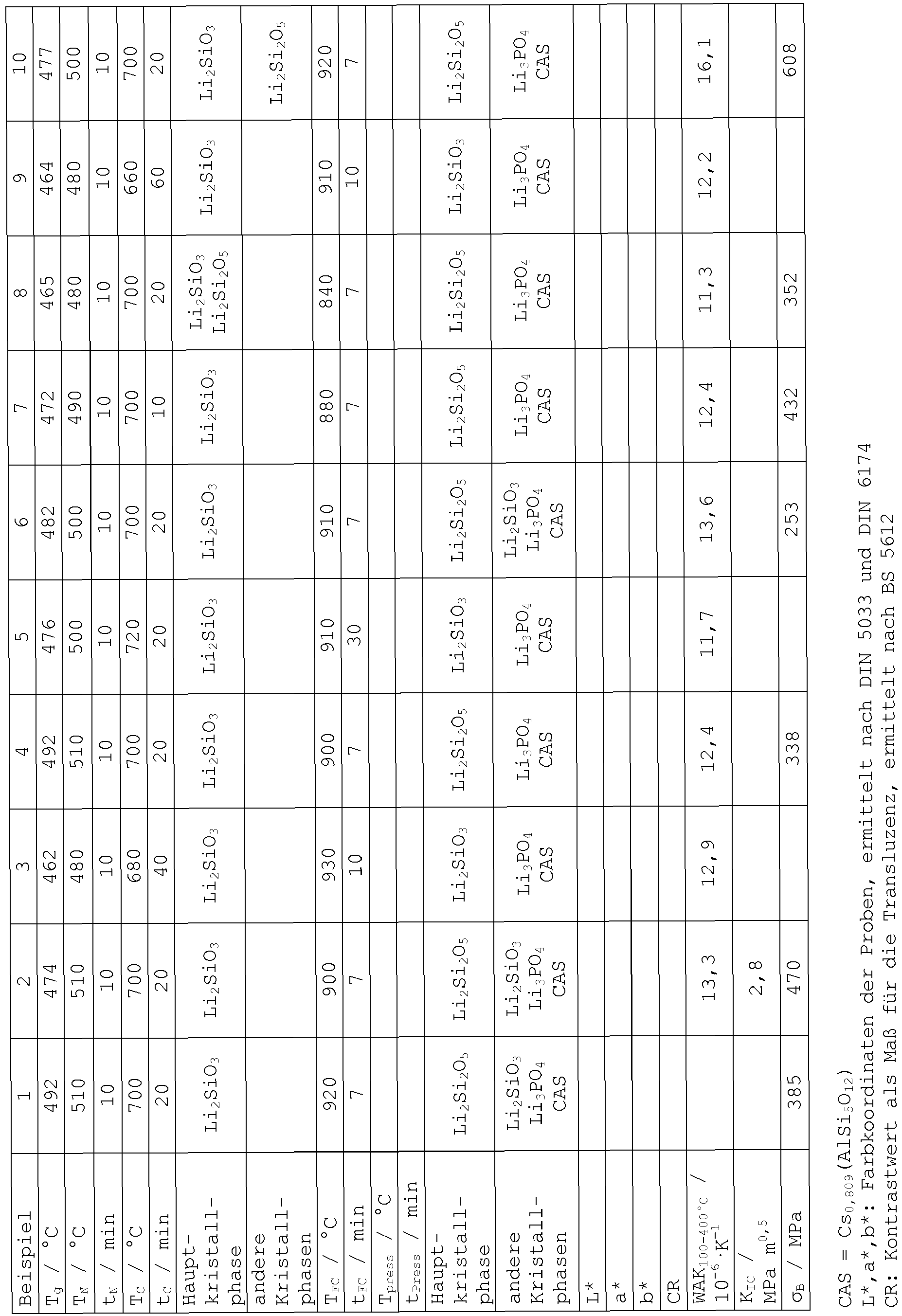

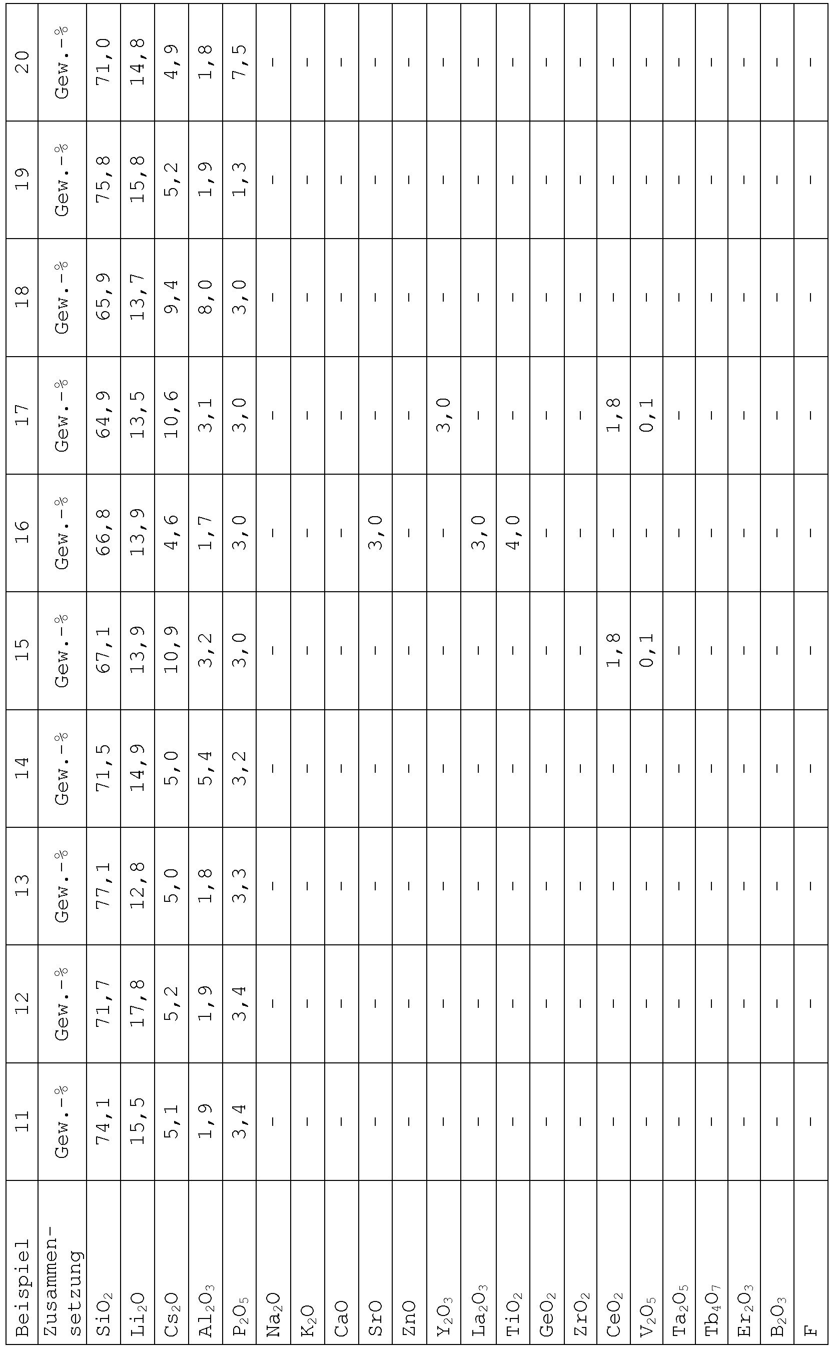

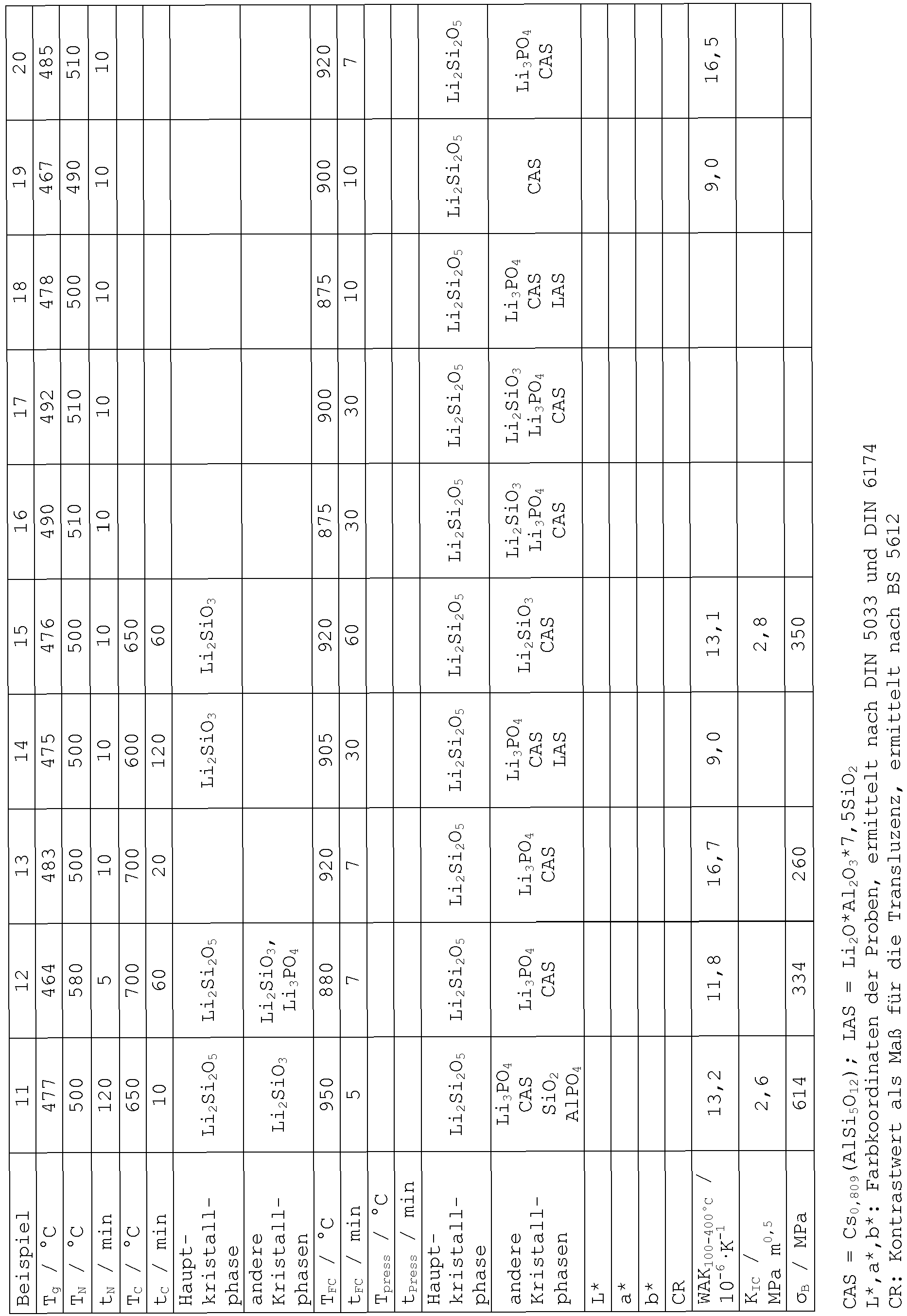

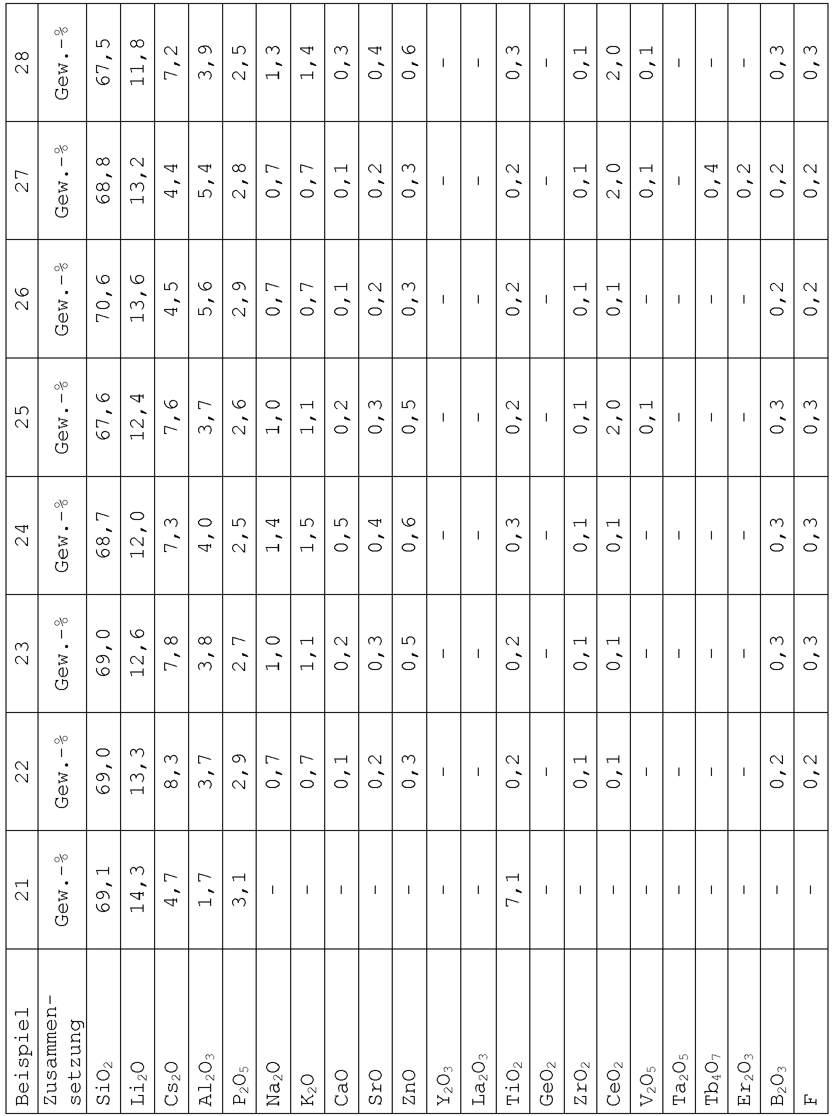

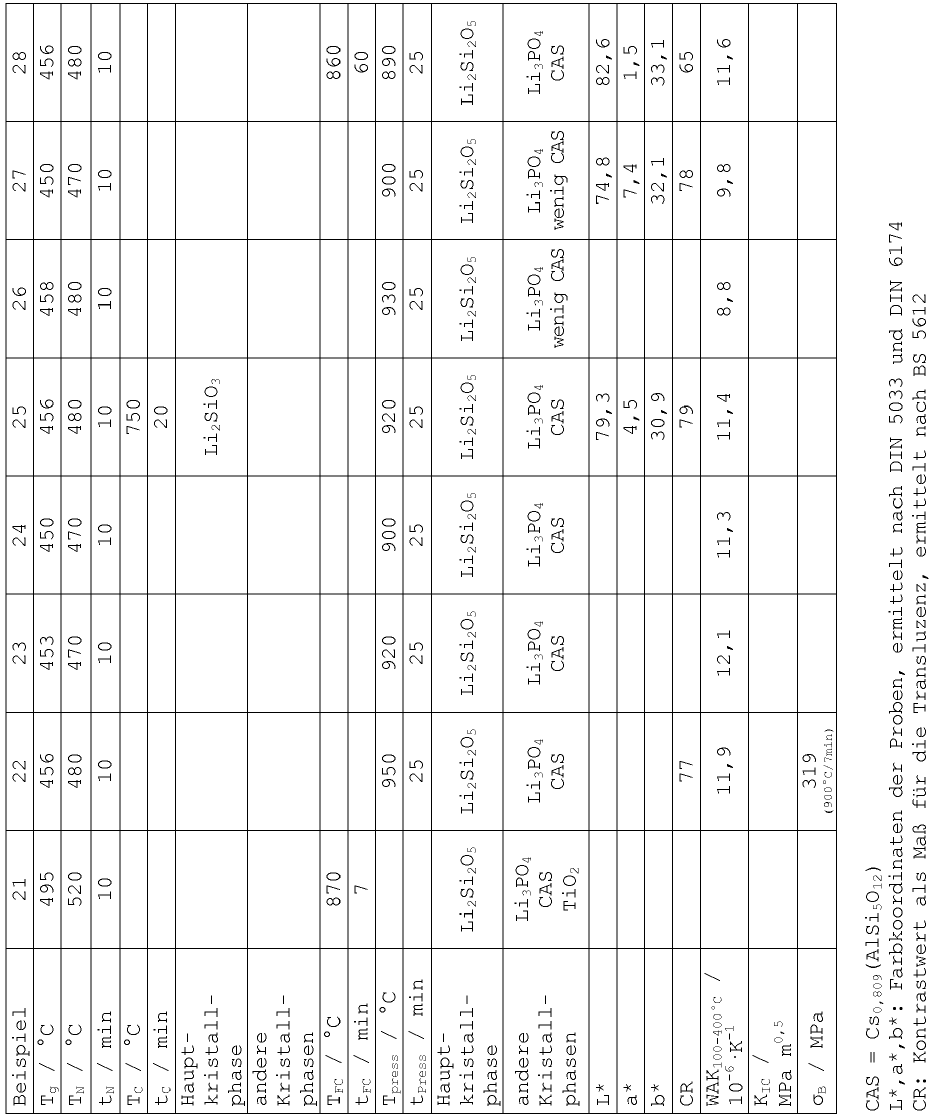

## Patentansprüche

1. Verwendung einer Lithiumsilikat-Glaskeramik oder eines Lithiumsilikatglases, die die folgenden Komponenten enthalten
| Komponente | Gew.-% | | |
|---|---|---|---|
| SiO₂ | 54,0 | bis | 78,0 |
| Li₂O | 11,8 | bis | 19,0 |
| Cs₂O | 4,4 | bis | 13,0 |
| Al₂O₃ | 1,7 | bis | 9,0 |
| P₂O₅ | 0,5 | bis | 9,0, |
zur Beschichtung eines Substrats ausgewählt aus Metallen und Legierungen.

2. Verwendung nach Anspruch 1, bei der die Lithiumsilikat-Glaskeramik oder das Lithiumsilikatglas mindestens eine und bevorzugt alle der folgenden Komponenten in den angegebenen Mengen enthalten
| Komponente | Gew.-% | | | |
|---|---|---|---|---|
| SiO₂ | 55,1 | bis | 77,1 | |
| Li₂O | 11,8 | bis | 17,8 | |
| Cs₂O | 4,4 | bis | 11,7 | |
| Al₂O₃ | 1,7 | bis | 8,0 | |
| P₂O₅ | 1,3 | bis | 7,5 | |
| ZrO₂ | 0 | bis | 4,5, | insbesondere 0 bis 4,0 |
| Übergangsmetalloxid | 0 | bis | 7,5, | insbesondere 0 bis 7,0, |
wobei das Übergangsmetalloxid ausgewählt ist aus der Gruppe bestehend aus Oxiden von Yttrium, Oxiden von Übergangsmetallen mit Ordnungszahl 41 bis 79 und Mischungen dieser Oxide.

3. Verwendung nach Anspruch 1 oder 2, bei der die Lithiumsilikat-Glaskeramik oder das Lithiumsilikatglas bis zu 70,0 Gew.-%, insbesondere 60,0 bis 70,0 Gew.-% und bevorzugt 64,0 bis 70,0 Gew.-% SiO₂ enthalten.

4. Verwendung nach einem der Ansprüche 1 bis 3, bei der die Lithiumsilikat-Glaskeramik oder das Lithiumsilikatglas 5,0 bis 10,0 Gew.-%, insbesondere 5,1 bis 8,0 Gew.-%, bevorzugt 5,5 bis 7,7 Gew.-% und besonders bevorzugt 6,1 bis 7,4 Gew.-% Cs₂O enthalten.

5. Verwendung nach einem der Ansprüche 1 bis 4, bei der die Lithiumsilikat-Glaskeramik oder das Lithiumsilikatglas 2,0 bis 6,0 Gew.-%, insbesondere 2,5 bis 5,0 und bevorzugt 3,0 bis 4,5 Gew.-% Al₂O₃ enthalten.

6. Verwendung nach einem der Ansprüche 1 bis 5, bei der die Lithiumsilikat-Glaskeramik oder das Lithiumsilikatglas weniger als 4,0 Gew.-%, insbesondere weniger als 3,5 Gew.-%, bevorzugt weniger als 3,0 Gew.-%, besonders bevorzugt weniger als 2,5 Gew.-% und am meisten bevorzugt weniger als 2,0 Gew.-% Na₂O und/oder K₂O enthalten.

7. Verwendung nach einem der Ansprüche 1 bis 6, bei der die Lithiumsilikat-Glaskeramik oder das Lithiumsilikatglas weniger als 2,5 Gew.-%, insbesondere weniger als 1,5 Gew.-%, bevorzugt weniger als 1,0 Gew.-%, besonders bevorzugt weniger als 0,5 Gew.-% Rb₂O enthalten und am meisten bevorzugt im Wesentlichen frei von Rb₂O sind.

8. Verwendung nach einem der Ansprüche 1 bis 7, bei der die Lithiumsilikat-Glaskeramik oder das Lithiumsilikatglas weniger als 3,8 Gew.-%, insbesondere weniger als 2,5 Gew.-%, bevorzugt weniger als 1,5 Gew.-%, besonders bevorzugt weniger als 0,5 Gew.-% BaO enthalten und am meisten bevorzugt im Wesentlichen frei von BaO sind.

9. Verwendung nach einem der Ansprüche 1 bis 8, bei der eine Lithiumsilikat-Glaskeramik verwendet wird, die Lithiummetasilikat als Hauptkristallphase enthält und vorzugsweise eine Biegefestigkeit im Bereich von etwa 180 bis 300 MPa und/oder eine Bruchzähigkeit, gemessen als K_{IC} Wert, von mindestens etwa 2,0 MPa·m^{0.5}, insbesondere mindestens etwa 2,3 MPa·m^{0.5} und vorzugsweise mindestens etwa 2,6 MPa·m^{0.5} aufweist.

10. Verwendung nach einem der Ansprüche 1 bis 9, bei der eine Lithiumsilikat-Glaskeramik verwendet wird, die Lithiumdisilikat als Hauptkristallphase enthält und vorzugsweise eine Biegefestigkeit im Bereich von etwa 400 bis 700 MPa und/oder eine Bruchzähigkeit, gemessen als K_{IC} Wert, von mindestens etwa 2,0 MPa·m^{0.5}, insbesondere mindestens etwa 2,3 MPa·m^{0.5} und vorzugsweise mindestens etwa 2,6 MPa·m^{0.5} aufweist.

11. Verwendung einer Lithiumsilikat-Glaskeramik, die die folgenden Komponenten enthält
| Komponente | Gew.-% | | |
|---|---|---|---|
| SiO₂ | 54,0 | bis | 78,0 |
| Li₂O | 11,0 | bis | 19,0 |
| Cs₂O | 4,0 | bis | 13,0 |
| Al₂O₃ | 1,0 | bis | 9,0 |
| P₂O₅ | 0,5 | bis | 9,0, |
zur Beschichtung eines Substrats ausgewählt aus Oxidkeramiken, Metallen und Legierungen,
wobei die Lithiumsilikat-Glaskeramik mindestens eine Cäsiumaluminosilikat-Kristallphase enthält, wobei das Cäsiumaluminosilikat vorzugsweise die Formel CsₓAlSi₅O₁₂ hat, wobei x 0,70 bis 0,90, insbesondere 0,75 bis 0,85, bevorzugt 0,80 bis 0,82 und besonders bevorzugt 0,808 bis 0,810 ist, und am meisten bevorzugt die Formel Cs_{0,809}AlSi₅O₁₂ hat.

12. Verwendung einer Lithiumsilikat-Glaskeramik, die die folgenden Komponenten enthält
| Komponente | Gew.-% | | |
|---|---|---|---|
| SiO₂ | 54,0 | bis | 78,0 |
| Li₂O | 11,0 | bis | 19,0 |
| Cs₂O | 4,0 | bis | 13,0 |
| Al₂O₃ | 1,0 | bis | 9, 0 |
| P₂O₅ | 0,5 | bis | 9,0, |
zur Beschichtung eines Substrats ausgewählt aus Oxidkeramiken, Metallen und Legierungen,
wobei die Lithiumsilikat-Glaskeramik mindestens eine Lithiumaluminosilikat-Kristallphase enthält, wobei das Lithiumaluminosilikat vorzugsweise die Formel Li₂O*Al₂O₃*7,5SiO₂ hat.

13. Verwendung nach einem der Ansprüche 1 bis 8, bei der ein Lithiumsilikatglas verwendet wird, wobei das Lithiumsilikatglas vorzugsweise Keime enthält, die zur Ausbildung von Lithiummetasilikat- und/oder Lithiumdisilikatkristallen geeignet sind.

14. Verwendung nach Anspruch 11 oder 12, bei der das Substrat eine Oxidkeramik und insbesondere eine Zirkonoxidkeramik ist.

15. Verwendung einer Lithiumsilikat-Glaskeramik oder eines Lithiumsilikatglases, die die folgenden Komponenten enthalten
| Komponente | Gew.-% | | |
|---|---|---|---|
| SiO₂ | 54,0 | bis | 78,0 |
| Li₂O | 11,0 | bis | 19,0 |
| Cs₂O | 4,0 | bis | 13,0 |
| Al₂O₃ | 1,0 | bis | 9, 0 |
| P₂O₅ | 0,5 | bis | 9,0, |
zur Beschichtung eines Substrats, wobei das Substrat eine Nichtedelmetall-Legierung ist.

16. Verwendung nach einem der Ansprüche 1 bis 15, bei der das Substrat eine Dentalrestauration und insbesondere eine Brücke, ein Inlay, ein Onlay, ein Veneer, ein Abutment, eine Teilkrone, eine Krone oder eine Schale ist.

17. Verfahren zur Beschichtung eines Substrats ausgewählt aus Metallen und Legierungen und insbesondere eines Substrats, das wie in Anspruch 15 oder 16 definiert ist, bei dem eine Lithiumsilikat-Glaskeramik oder ein Lithiumsilikatglas auf das Substrat aufgebracht werden, wobei die Glaskeramik wie in einem der Ansprüche 1 bis 12 definiert ist und das Glas wie in einem der Ansprüche 1 bis 8 definiert ist.

18. Verfahren nach Anspruch 17, bei dem die Lithiumsilikat-Glaskeramik oder das Lithiumsilikatglas durch Aufsintern und bevorzugt durch Aufpressen auf das Substrat aufgebracht werden.

19. Verfahren nach Anspruch 17, bei dem die Lithiumsilikat-Glaskeramik oder das Lithiumsilikatglas durch Fügen auf das Substrat aufgebracht werden.

20. Verfahren nach Anspruch 19, bei dem die Lithiumsilikat-Glaskeramik oder das Lithiumsilikatglas vor dem Fügen durch maschinelle Bearbeitung oder durch Heißpressen zu einer gewünschten Geometrie verformt werden.

21. Verfahren nach einem der Ansprüche 17 bis 20, bei dem eine Beschichtung erhalten wird, die eine Lithiumsilikat-Glaskeramik umfasst, welche Lithiummetasilikat als Hauptkristallphase enthält, und vorzugsweise eine Biegefestigkeit im Bereich von etwa 180 bis 300 MPa und/oder eine Bruchzähigkeit, gemessen als K_{IC} Wert, von mindestens etwa 2,0 MPa·m^{0.5}, insbesondere mindestens etwa 2,3 MPa·m^{0.5} und vorzugsweise mindestens etwa 2,6 MPa·m^{0.5} aufweist.

22. Verfahren nach einem der Ansprüche 17 bis 20, bei dem eine Beschichtung erhalten wird, die eine Lithiumsilikat-Glaskeramik umfasst, welche Lithiumdisilikat als Hauptkristallphase enthält, und vorzugsweise eine Biegefestigkeit im Bereich von etwa 400 bis 700 MPa und/oder eine Bruchzähigkeit, gemessen als K_{IC} Wert, von mindestens etwa 2,0 MPa·m^{0.5}, insbesondere mindestens etwa 2,3 MPa·m^{0.5} und vorzugsweise mindestens etwa 2,6 MPa·m^{0.5} aufweist.

23. Verfahren nach Anspruch 21 oder 22, bei dem die Lithiumsilikat-Glaskeramik mindestens eine Cäsiumaluminosilikat-Kristallphase enthält, wobei das Cäsiumaluminosilikat vorzugsweise die Formel CsₓAlSi₅O₁₂ hat, wobei x 0,70 bis 0,90, insbesondere 0,75 bis 0,85, bevorzugt 0,80 bis 0,82 und besonders bevorzugt 0,808 bis 0,810 ist, und am meisten bevorzugt die Formel Cs_{0,809}AlSi₅O₁₂ hat, und bevorzugt ferner mindestens eine Lithiumaluminosilikat-Kristallphase enthält, wobei das Lithiumaluminosilikat vorzugsweise die Formel Li₂O*Al₂O₃*7,5SiO₂ hat.

24. Verbundwerkstoff, der eine Lithiumsilikat-Glaskeramik oder ein Lithiumsilikatglas auf einem Substrat ausgewählt aus Metallen und Legierungen und insbesondere auf einem Substrat umfasst, das wie in Anspruch 15 oder 16 definiert ist, wobei die Glaskeramik wie in einem der Ansprüche 1 bis 12 definiert ist und das Glas wie in einem der Ansprüche 1 bis 8 definiert ist.

25. Lithiumsilikat-Glaskeramik, die wie in einem der Ansprüche 11 oder 12 definiert ist.

26. Lithiumsilikat-Glaskeramik, die wie in einem der Ansprüche 1 bis 12 definiert ist und die die folgenden Komponenten in den angegebenen Mengen enthält
| Komponente | Gew.-% | | | | | | |
|---|---|---|---|---|---|---|---|
| SiO₂ | 54,0 | bis | 78,0, | insbesondere | 55,1 | bis | 77,1 |
| Li₂O | 11,8 | bis | 19,0, | insbesondere | 11,8 | bis | 17,8 |
| Cs₂O | 4,6 | bis | 13,0, | insbesondere | 4,7 | bis | 11,7 |
| Al₂O₃ | 1,7 | bis | 9,0, | insbesondere | 1,7 | bis | 8,0 |
| P₂O₅ | 0,5 | bis | 9,0, | insbesondere | 1,3 | bis | 7,5 |
| Rb₂O | 0 | bis | 7,0, | insbesondere | 0 | bis | 5,0 |
| BaO | 0 | bis | 3,7, | insbesondere | 0 | bis | 3,0 |
| ZrO₂ | 0 | bis | 4,5, | insbesondere | 0 | bis | 4,0 |
| Übergangsmetalloxid | 0 | bis | 7,5, | insbesondere | 0 | bis | 7,0, |
wobei das Übergangsmetalloxid ausgewählt ist aus der Gruppe bestehend aus Oxiden von Yttrium, Oxiden von Übergangsmetallen mit Ordnungszahl 41 bis 79 und Mischungen dieser Oxide.

27. Lithiumsilikatglas, das die Komponenten der Glaskeramik nach Anspruch 26 enthält.

## Claims

1. Use of a lithium silicate glass ceramic or a lithium silicate glass which comprise the following components
| Component | wt.-% |
|---|---|
| SiO₂ | 54.0 to 78.0 |
| Li₂O | 11.8 to 19.0 |
| Cs₂O | 4.4 to 13.0 |
| Al₂O₃ | 1.7 to 9.0 |
| P₂O₅ | 0.5 to 9.0, |
for coating a substrate selected from metals and alloys.

2. Use according to claim 1, in which the lithium silicate glass ceramic or the lithium silicate glass comprise at least one and preferably all of the following components in the given amounts
| Component | wt.-% |
|---|---|
| SiO₂ | 55.1 to 77.1 |
| Li₂O | 11.8 to 17.8 |
| Cs₂O | 4.4 to 11.7 |
| Al₂O₃ | 1.7 to 8.0 |
| P₂O₅ | 1.3 to 7.5 |
| ZrO₂ | 0 to 4.5, in particular 0 to 4.0 |
| Transition metal oxide | 0 to 7.5, in particular 0 to 7.0, |
wherein the transition metal oxide is selected from the group consisting of oxides of yttrium, oxides of transition metals with an atomic number from 41 to 79 and mixtures of these oxides.

3. Use according to claim 1 or 2, in which the lithium silicate glass ceramic or the lithium silicate glass comprise up to 70.0 wt.-%, in particular 60.0 to 70.0 wt.-% and preferably 64.0 to 70.0 wt.-% SiO₂.

4. Use according to any one of claims 1 to 3, in which the lithium silicate glass ceramic or the lithium silicate glass comprise 5.0 to 10.0 wt.-%, in particular 5.1 to 8.0 wt.-%, preferably 5.5 to 7.7 wt.-% and particularly preferably 6.1 to 7.4 wt.-% Cs₂O.

5. Use according to any one of claims 1 to 4, in which the lithium silicate glass ceramic or the lithium silicate glass comprise 2.0 to 6.0 wt.-%, in particular 2.5 to 5.0 and preferably 3.0 to 4.5 wt.-% Al₂O₃.

6. Use according to any one of claims 1 to 5, in which the lithium silicate glass ceramic or the lithium silicate glass comprise less than 4.0 wt.-%, in particular less than 3.5 wt.-%, preferably less than 3.0 wt.-%, particularly preferably less than 2.5 wt.-% and most preferably less than 2.0 wt.-% Na₂O and/or K₂O.

7. Use according to any one of claims 1 to 6, in which the lithium silicate glass ceramic or the lithium silicate glass comprise less than 2.5 wt.-%, in particular less than 1.5 wt.-%, preferably less than 1.0 wt.-%, particularly preferably less than 0.5 wt.-% Rb₂O and is most preferably substantially free of Rb₂O.

8. Use according to any one of claims 1 to 7, in which the lithium silicate glass ceramic or the lithium silicate glass comprise less than 3.8 wt.-%, in particular less than 2.5 wt.-%, preferably less than 1.5 wt.-%, particularly preferably less than 0.5 wt.-% BaO and is most preferably substantially free of BaO.

9. Use according to any one of claims 1 to 8, in which a lithium silicate glass ceramic is used which comprises lithium metasilicate as main crystal phase and preferably has a bending strength in the range of about 180 to 300 MPa and/or a fracture toughness, measured as K_{IC} value, of at least about 2.0 MPa·m^{0.5}, in particular at least about 2.3 MPa·m^{0.5} and preferably at least about 2.6 MPa·m^{0.5}.

10. Use according to any one of claims 1 to 9, in which a lithium silicate glass ceramic is used which comprises lithium disilicate as main crystal phase and preferably has a bending strength in the range of about 400 to 700 MPa and/or a fracture toughness, measured as K_{IC} value, of at least about 2.0 MPa·m^{0.5}, in particular at least about 2.3 MPa·m^{0.5} and preferably at least about 2.6 MPa·m^{0.5}.

11. Use of a lithium silicate glass ceramic which comprises the following components
| Component | wt.-% |
|---|---|
| SiO₂ | 54.0 to 78.0 |
| Li₂O | 11.0 to 19.0 |
| Cs₂O | 4.0 to 13.0 |
| Al₂O₃ | 1.0 to 9.0 |
| P₂O₅ | 0.5 to 9.0, |
for coating a substrate selected from oxide ceramics, metals and alloys,
wherein the lithium silicate glass ceramic comprises at least one caesium aluminosilicate crystal phase, wherein the caesium aluminosilicate preferably has the formula CsₓAlSi₅O₁₂, wherein x is 0.70 to 0.90, in particular 0.75 to 0.85, preferably 0.80 to 0.82 and particularly preferably 0.808 to 0.810, and most preferably has the formula Cs_{0.809}AlSi₅O₁₂.

12. Use of a lithium silicate glass ceramic which comprises the following components
| Component | wt.-% |
|---|---|
| SiO₂ | 54.0 to 78.0 |
| Li₂O | 11.0 to 19.0 |
| Cs₂O | 4.0 to 13.0 |
| Al₂O₃ | 1.0 to 9.0 |
| P₂O₅ | 0.5 to 9.0, |
for coating a substrate selected from oxide ceramics, metals and alloys,
wherein the lithium silicate glass ceramic comprises at least one lithium aluminosilicate crystal phase, wherein the lithium aluminosilicate preferably has the formula Li₂O*Al₂O₃*7.5SiO₂.

13. Use according to any one of claims 1 to 8, in which a lithium silicate glass is used, wherein the lithium silicate glass preferably comprises nuclei which are suitable for forming lithium metasilicate and/or lithium disilicate crystals.

14. Use according to claim 11 or 12, in which the substrate is an oxide ceramic and in particular a zirconium oxide ceramic.

15. Use of a lithium silicate glass ceramic or a lithium silicate glass which comprise the following components
| Component | wt.-% |
|---|---|
| SiO₂ | 54.0 to 78.0 |
| Li₂O | 11.0 to 19.0 |
| Cs₂O | 4.0 to 13.0 |
| Al₂O₃ | 1.0 to 9.0 |
| P₂O₅ | 0.5 to 9.0, |
for coating a substrate which substrate is a a non-precious metal alloy.

16. Use according to any one of claims 1 to 15, in which the substrate is a dental restoration and in particular a bridge, an inlay, an onlay, a veneer, an abutment, a partial crown, a crown or a facet.

17. Process for coating a substrate selected from metals and alloys, and in particular a substrate which is as defined in claim 15 or 16, in which a lithium silicate glass ceramic or a lithium silicate glass is applied to the substrate, wherein the glass ceramic is as defined in any one of claims 1 to 12 and the glass is as defined in any one of claims 1 to 8.

18. Process according to claim 17, in which the lithium silicate glass ceramic or the lithium silicate glass is applied to the substrate by sintering and preferably by pressing-on.

19. Process according to claim 17, in which the lithium silicate glass ceramic or the lithium silicate glass is applied to the substrate by joining.

20. Process according to claim 19, in which the lithium silicate glass ceramic or the lithium silicate glass is shaped to a desired geometry by machining or by hot pressing before joining.

21. Process according to any one of claims 17 to 20, in which a coating is obtained which comprises a lithium silicate glass ceramic that comprises lithium metasilicate as main crystal phase, and which preferably has a bending strength in the range of about 180 to 300 MPa and/or a fracture toughness, measured as K_{IC} value, of at least about 2.0 MPa·m^{0.5}, in particular at least about 2.3 MPa·m^{0.5} and preferably at least about 2.6 MPa·m^{0.5}.

22. Process according to any one of claims 17 to 20, in which a coating is obtained which comprises a lithium silicate glass ceramic that comprises lithium disilicate as main crystal phase, and which preferably has a bending strength in the range of about 400 to 700 MPa and/or a fracture toughness, measured as K_{IC} value, of at least about 2.0 MPa·m^{0.5}, in particular at least about 2.3 MPa·m^{0.5} and preferably at least about 2.6 MPa·m^{0.5}.

23. Process according to claim 21 or 22, in which the lithium silicate glass ceramic comprises at least one caesium aluminosilicate crystal phase, wherein the caesium aluminosilicate preferably has the formula CsₓAlSi₅O₁₂, wherein x is 0.70 to 0.90, in particular 0.75 to 0.85, preferably 0.80 to 0.82 and particularly preferably 0.808 to 0.810, and most preferably has the formula Cs_{0.809}AlSi₅O₁₂, and preferably further comprises at least one lithium aluminosilicate crystal phase, wherein the lithium aluminosilicate preferably has the formula Li₂O*Al₂O₃*7.5SiO₂.

24. Composite material which comprises a lithium silicate glass ceramic or a lithium silicate glass on a substrate selected from metals and alloys, and in particular on a substrate which is as defined in claim 15 or 16, wherein the glass ceramic is as defined in any one of claims 1 to 12 and the glass is as defined in any one of claims 1 to 8.

25. Lithium silicate glass ceramic, which is as defined in any one of claims 11 or 12.

26. Lithium silicate glass ceramic, which is as defined in any one of claims 1 to 12 and which comprises the following components in the given amounts
| Component | wt.-% |
|---|---|
| SiO₂ | 54.0 to 78.0, in particular 55.1 to 77.1 |
| Li₂O | 11.8 to 19.0, in particular 11.8 to 17.8 |
| Cs₂O | 4.6 to 13.0, in particular 4.7 to 11.7 |
| Al₂O₃ | 1.7 to 9.0, in particular 1.7 to 8.0 |
| P₂O₅ | 0.5 to 9.0, in particular 1.3 to 7.5 |
| Rb₂O | 0 to 7.0, in particular 0 to 5.0 |
| BaO | 0 to 3.7, in particular 0 to 3.0 |
| ZrO₂ | 0 to 4.5, in particular 0 to 4.0 |
| Transition metal oxide | 0 to 7.5, in particular 0 to 7.0, |
wherein the transition metal oxide is selected from the group consisting of oxides of yttrium, oxides of transition metals with an atomic number from 41 to 79 and mixtures of these oxides.

27. Lithium silicate glass, which comprises the components of the glass ceramic according to claim 26.

## Revendications

1. Utilisation d'une vitrocéramique à base de silicate de lithium ou d'un verre à base de silicate de lithium, qui contiennent les composants suivants
| Composant | % en poids |
|---|---|
| SiO₂ | 54,0 à 78,0 |
| Li₂O | 11,8 à 19,0 |
| Cs₂O | 4,4 à 13,0 |
| Al₂O₃ | 1,7 à 9,0 |
| P₂O₅ | 0,5 à 9,0<, |
pour le revêtement d'un support choisi parmi les métaux et alliages.

2. Utilisation selon la revendication 1, dans laquelle la vitrocéramique à base de silicate de lithium ou le verre à base de silicate de lithium contiennent au moins l'un et de préférence la totalité des composants suivants en les quantités indiquées
| Composant | % en poids |
|---|---|
| SiO₂ | 55,1 à 77,1 |
| Li₂O | 11,8 à 17,8 |
| Cs₂O | 4,4 à 11,7 |
| Al₂O₃ | 1,7 à 8,0 |
| P₂O₅ | 1,3 à 7,5, |
| ZrO₂ | 0 à 4,5, en particulier 0 à 4,0 |
| oxyde de métal de transition | 0 à 7,5, en particulier 0 à 7,0, |
l'oxyde de métal de transition étant choisi dans l'ensemble constitué par les oxydes d'yttrium, les oxydes de métaux de transition de nombre atomique 41 à 79 et les mélanges de ces oxydes.

3. Utilisation selon la revendication 1 ou 2, dans laquelle la vitrocéramique à base de silicate de lithium ou le verre à base de silicate de lithium contiennent jusqu'à 70,0 % en poids, en particulier 60,0 à 70 % en poids et de préférence 64,0 à 70,0 % en poids de SiO₂.

4. Utilisation selon l'une quelconque des revendications 1 à 3, dans laquelle la vitrocéramique à base de silicate de lithium ou le verre à base de silicate de lithium contiennent 5,0 à 10,0 % en poids, en particulier 5,1 à 8,0 % en poids, de préférence 5,5 à 7,7 % en poids et de façon particulièrement préférée 6,1 à 7,4 % en poids de Cs₂O.

5. Utilisation selon l'une quelconque des revendications 1 à 4, dans laquelle la vitrocéramique à base de silicate de lithium ou le verre à base de silicate de lithium contiennent 2,0 à 6,0 % en poids, en particulier 2,5 à 5,0 et de préférence 3,0 à 4,5 % en poids d'Al₂O₃.

6. Utilisation selon l'une quelconque des revendications 1 à 5, dans laquelle la vitrocéramique à base de silicate de lithium ou le verre à base de silicate de lithium contiennent moins de 4,0 % en poids, en particulier moins de 3,5 % en poids, de préférence moins de 3,0 % en poids, de façon particulièrement préférée moins de 2,5 % en poids et de façon tout particulièrement préférée moins de 2,0 % en poids de Na₂O et/ou K₂O.

7. Utilisation selon l'une quelconque des revendications 1 à 6, dans laquelle la vitrocéramique à base de silicate de lithium ou le verre à base de silicate de lithium contiennent moins de 2,5 % en poids, en particulier moins de 1,5 % en poids, de préférence moins de 1,0 % en poids, de façon particulièrement préférée moins de 0,5 % en poids de Rb₂O et de façon tout particulièrement préférée sont pratiquement exempts de Rb₂O.

8. Utilisation selon l'une quelconque des revendications 1 à 7, dans laquelle la vitrocéramique à base de silicate de lithium ou le verre à base de silicate de lithium contiennent moins de 3,8 % en poids, en particulier moins de 2,5 % en poids, de préférence moins de 1,5 % en poids, de façon particulièrement préférée moins de 0,5 % en poids de BaO et de façon tout particulièrement préférée sont pratiquement exempts de BaO.

9. Utilisation selon l'une quelconque des revendications 1 à 8, dans laquelle on utilise une vitrocéramique à base de silicate de lithium qui contient du métasilicate de lithium en tant que phase cristalline principale et présente de préférence une résistance à la flexion dans la plage d'environ 180 à 300 MPa et/ou une résistance à la rupture, mesurée en tant que valeur K_{IC}, d'au moins environ 2,0 MPa.m^{0,5}, en particulier d'au moins environ 2,3 MPa.m^{0,5} et de préférence d'au moins environ 2,6 MPa.m^{0,5}.

10. Utilisation selon l'une quelconque des revendications 1 à 9, dans laquelle on utilise une vitrocéramique à base de silicate de lithium qui contient du disilicate de lithium en tant que phase cristalline principale et présente de préférence une résistance à la flexion dans la plage d'environ 400 à 700 MPa et/ou une résistance à la rupture, mesurée en tant que valeur K_{IC}, d'au moins environ 2,0 MPa.m^{0,5}, en particulier d'au moins environ 2,3 MPa.m^{0,5} et de préférence d'au moins environ 2,6 MPa.m^{0,5}.

11. Utilisation d'une vitrocéramique à base de silicate de lithium, qui contient les composants suivants
| Composant | % en poids |
|---|---|
| SiO₂ | 54,0 à 78,0 |
| Li₂O | 11,0 à 19,0 |
| Cs₂O | 4,0 à 13,0 |
| Al₂O₃ | 1,0 à 9,0 |
| P₂O₅ | 0,5 à 9,0, |
pour le revêtement d'un support choisi parmi les céramiques à base d'oxydes, les métaux et alliages.
dans laquelle la vitrocéramique à base de silicate de lithium contient au moins une phase cristalline d'aluminosilicate de césium, l'aluminosilicate de césium ayant de préférence la formule CsₓAlSi₅O₁₂, où x vaut de 0,70 à 0,90, en particulier de 0,75 à 0,85, de préférence de 0,80 à 0,82 et de façon particulièrement préférée de 0,808 à 0,810, et de façon tout particulièrement préférée ayant la formule Cs_{0,809}AlSi₅O₁₂.

12. Utilisation d'une vitrocéramique à base de silicate de lithium, qui contient les composants suivants
| Composant | % en poids |
|---|---|
| SiO₂ | 54,0 à 78,0 |
| Li₂O | 11,0 à 19,0 |
| Cs₂O | 4,0 à 13,0 |
| Al₂O₃ | 1,0 à 9,0 |
| P₂O₅ | 0,5 à 9,0, |
pour le revêtement d'un support choisi parmi les céramiques à base d'oxydes, les métaux et alliages,
dans laquelle la vitrocéramique à base de silicate de lithium contient au moins une phase cristalline d'aluminosilicate de lithium, l'aluminosilicate de lithium ayant de préférence la formule Li₂O^{∗}Al₂O₃^{∗}7,5SiO₂.

13. Utilisation selon l'une quelconque des revendications 1 à 8, dans laquelle on utilise un verre à base de silicate de lithium, le verre à base de silicate de lithium contenant de préférence des germes qui sont appropriés à la formation de cristaux de métasilicate de lithium et/ou de cristaux de disilicate de lithium.

14. Utilisation selon la revendication 11 ou 12, dans laquelle le support est une céramique à base d'oxyde et en particulier une céramique à base d'oxyde de zirconium.

15. Utilisation d'une vitrocéramique à base de silicate de lithium ou d'un verre à base de silicate de lithium, qui contiennent les composants suivants
| Composant | % en poids |
|---|---|
| SiO₂ | 54,0 à 78,0 |
| Li₂O | 11,0 à 19,0 |
| Cs₂O | 4,0 à 13,0 |
| Al₂O₃ | 1,0 à 9,0 |
| P₂O₅ | 0,5 à 9,0, |
pour le revêtement d'un support, le support étant un alliage de métaux non précieux.

16. Utilisation selon l'une quelconque des revendications 1 à 15, dans laquelle le support est une restauration dentaire et en particulier un bridge, un inlay, un onlay, un placage, un pilier, une couronne partielle, une couronne ou une facette.

17. Procédé pour le revêtement d'un support choisi parmi les métaux et alliages et en particulier d'un support qui est tel que défini dans la revendication 15 ou 16, dans lequel on applique sur le support une vitrocéramique à base de silicate de lithium ou un verre à base de silicate de lithium, la vitrocéramique étant telle que définie dans l'une quelconque des revendications 1 à 12 et le verre étant tel que défini dans l'une quelconque des revendications 1 à 8.

18. Procédé selon la revendication 17, dans lequel on applique sur le support la vitrocéramique à base de silicate de lithium ou le verre à base de silicate de lithium par application par frittage et de préférence par application par pressage.

19. Procédé selon la revendication 17, dans lequel on applique sur le support la vitrocéramique à base de silicate de lithium ou le verre à base de silicate de lithium par assemblage.

20. Procédé selon la revendication 19, dans lequel avant l'assemblage on met en forme à une géométrie désirée la vitrocéramique à base de silicate de lithium ou le verre à base de silicate de lithium par usinage mécanique ou par pressage à chaud.

21. Procédé selon l'une quelconque des revendications 17 à 20, dans lequel on obtient un revêtement qui comprend une vitrocéramique à base de silicate de lithium, qui contient du métasilicate de lithium en tant que phase cristalline principale et présente de préférence une résistance à la flexion dans la plage d'environ 180 à 300 MPa et/ou une résistance à la rupture, mesurée en tant que valeur K_{IC}, d'au moins environ 2,0 MPa.m^{0,5} en particulier d'au moins environ 2,3 MPa.m^{0,5} et de préférence d'au moins environ 2,6 MPa.m^{0,5}.

22. Procédé selon l'une quelconque des revendications 17 à 20, dans lequel on obtient un revêtement qui comprend une vitrocéramique à base de silicate de lithium, qui contient du disilicate de lithium en tant que phase cristalline principale et présente de préférence une résistance à la flexion dans la plage d'environ 400 à 700 MPa et/ou une résistance à la rupture, mesurée en tant que valeur K_{IC}, d'au moins environ 2,0 MPa.m^{0,5} en particulier d'au moins environ 2,3 MPa.m^{0,5} et de préférence d'au moins environ 2,6 MPa.m^{0,5}.

23. Procédé selon la revendications 21 ou 22, dans lequel la vitrocéramique à base de silicate de lithium contient au moins une phase cristalline d'aluminosilicate de césium, l'aluminosilicate de césium ayant de préférence la formule CsₓAlSi₅O₁₂, où x vaut de 0,70 à 0,90, en particulier de 0,75 à 0,85, de préférence de 0,80 à 0,82 et de façon particulièrement préférée de 0,808 à 0,810, et de façon tout particulièrement préférée ayant la formule Cs_{0,809}AlSi₅O₁₂, et de préférence contient en outre au moins une phase cristalline d'aluminosilicate de lithium, l'aluminosilicate de lithium ayant de préférence la formule Li₂O^{∗}Al_{2O3}^{∗}7,5SiO₂.

24. Matériau composite qui comprend une vitrocéramique à base de silicate de lithium ou un verre à base de silicate de lithium sur un support choisi parmi les métaux ou alliages et en particulier sur un support qui est tel que défini dans la revendication 15 ou 16, la vitrocéramique étant telle que définie dans l'une quelconque des revendications 1 à 12 et le verre étant tel que défini dans l'une quelconque des revendications 1 à 8.

25. Vitrocéramique à base de silicate de lithium, qui est telle que définie dans l'une quelconque des revendications 11 et 12.

26. Vitrocéramique à base de silicate de lithium, qui est telle que définie dans l'une quelconque des revendications 1 à 12 et qui contient les composants suivants en les quantités indiquées
| Composant | % en poids |
|---|---|
| SiO₂ | 54,0 à 78,0, en particulier 55,1 à 77,1 |
| Li₂O | 11,8 à 19,0, en particulier 11,8 à 17,8 |
| Cs₂O | 4,6 à 13,0, en particulier 4,7 à 11,7 |
| Al₂O₃ | 1,7 à 9,0, en particulier 1,7 à 8,0 |
| P₂O₅ | 0,5 à 9,0, en particulier 1,3 à 7,5 |
| Rb₂O | 0 à 7,0, en particulier 0 à 5,0 |
| BaO | 0 à 3,7, en particulier 0 à 3,0 |
| ZrO₂ | 0 à 4,5, en particulier 0 à 4,0 |
| oxyde de métal de transition | 0 à 7,5, en particulier 0 à 7,0 |
l'oxyde de métal de transition étant choisi dans l'ensemble constitué par les oxydes d'yttrium, les oxydes de métaux de transition de nombre atomique 41 à 79 et les mélanges de ces oxydes.

27. Verre à base de silicate de lithium, qui contient les composants de la vitrocéramique selon la revendication 26.
